# EUROPEAN PATENT SPECIFICATION

(11) **EP 0 616 520 B1**
(45) Date of publication and mention of the grant of the patent: **29.05.1996**
(21) Application number: 93901384.3
(22) Date of filing: 10.12.1992
(51) Int. Cl.: A61K 7/16

(54) **PHYTATE-ANTIMICROBIAL COMPOSITIONS IN ORAL CARE PRODUCTS**
PHYTAT-ANTIMICROBIELLE ZUSAMMENSETZUNGEN IN MUNDPFLEGEMITTELN
COMPOSITIONS ANTIMICROBIENNES AU PHYTATE POUR PRODUITS DE SOINS BUCCO-DENTAIRES

(30) Priority: 10.12.1991 US 806070
(43) Date of publication of application: 28.09.1994
(73) Proprietor: THE DOW CHEMICAL COMPANY, Midland, Michigan 48640 (US)
(72) Inventor: GARLICH, Joseph, R., Lake Jackson, TX 77566 (US); MASTERSON, Tipton, T., Lake Jackson, TX 77566 (US); FRANK, R., Keith, Lake Jackson, TX 77566 (US)
(74) Representative: Burford, Anthony Frederick
(86) International application number: PCT/US92/10665
(87) International publication number: WO 93/11740

(56) References cited:
- EP-A- 0 329 069
- US-A- 4 263 276
- US-A- 4 305 928
- US-A- 4 335 102
- Patent Abstract of Japan, Vol 16, No 207, abstract of JP, A, 4-36229 (SUNSTAR INC.), 6 February 1992 (06.02.92)

## Description

The present invention relates to oral compositions containing an anticalculus or an antiplaque/antigingivitis agent.

"Oral composition" means a composition for topical applications to the oral cavity to clean and care for the teeth as well as the oral cavity surfaces. Representatives of such compositions are oral hygiene products and dentifrices such as mouthwashes or rinses, toothpaste, dental gels, tooth powder, chewing gum, lozenges, and similar products. In addition to cleaning teeth to remove dental plaque, the function of oral hygiene preparations is to stop the formation of dental calculus, to prevent dental disorders such as caries, periodontitis and gingivitis, and also to eliminate halitosis.

Dental calculus, or tartar as it is sometimes called, is a hard mineralized material which forms on teeth that consists of inorganic and organic components. The inorganic portion is largely calcium and orthophosphate arranged in a crystal lattice called hydroxyapatite (HAP). The organic portion is derived mainly from microorganisms (i.e., bacteria, yeast, etc.) as well as epithelial cells, white blood cells and food debris.

Formation of dental calculus occurs in two steps. In the first step, plaque is deposited on the teeth. "Plaque" consists of inorganic and organic components derived from saliva, food and bacteria which are present in the oral cavity. Most of the plaque consists of dead and living bacteria surrounded by a gel-like matrix derived from the bacteria and saliva. In the second phase, plaque undergoes calcification to form dental calculus. Initially, amorphous deposits of calcium phosphate begins to appear on and within the matrix of the dental plaque. As the aggregates of calcium phosphate become sufficiently closely packed together, they crystallize to form HAP. The amorphous calcium phosphate, although related to hydroxyapatite, differs from it in crystal structure, particle morphology and stoichiometry.

In addition to being an integral step for the formation of calculus, consequences of the presence of plaque include gingivitis, periodontitis, tooth decay (dental caries) and denture associated problems. Inhibition of oral bacteria involved in the formation of plaque by antimicrobials or antiseptic agents is one means to retard the formation of plaque, thus aiding in preventing or controlling the formation of calculus and other plaque related diseases; see, for example, P. S. Hull, *J. Clinical Periodontology,* 7, 431-442 (1980). Examples of antiseptic agents include bisbiguanides, such as chlorhexidine and alexidine, and numerous antibacterially active quaternary ammonium compounds, such as cetylpyridinium chloride or the quaternary ammonium compounds described in US-A-3,369,046 and US-A-4,820,507; and quaternary ammonium organosiloxane compounds described in US-A-4,161,518.

Although the quaternary ammonium compounds are rapidly adsorbed to the tooth surfaces, they exhibit only a moderate degree of efficacy as antiplaque and antigingivitis agents as they are rapidly released from the tooth surface and thus retained in the oral cavity for only a short period of time. Chlorhexidine has been the most successful antiplaque agent as it is believed to bind to the oral mucosa and is thus retained in the oral cavity for a longer period of time than quaternary ammonium compounds. The use of chlorhexidine in oral preparations however, suffers from the following disadvantages: (1) a prolonged bitter after taste lasting up to several hours; (2) after prolonged use stains are produced on the teeth, tongue, gums, oral mucosa and dental restorations; and (3) production of local irritation of the oral mucosa and tongue.

Inhibition of crystalline HAP formation is usually achieved by compounds which sorb onto a growing crystal and disrupt crystal growth. It is well known in the prior art that water soluble hexametaphosphates, tripolyphosphates and pyrophosphates and the like, are effective calcium and magnesium ion sequestrants and/or chelating agents. See, for example, US-A-3,488,419 which discloses oral compositions containing polyphosphate and US-A-4,215,105 which discloses oral compositions containing phosphonoacetic acid. However, as described in US-A-4,627,977, the effectiveness of polyphosphates as anticalculus agents has been limited because they are significantly hydrolyzed by salivary enzymes (phosphatases) to orthophosphates which are ineffective as inhibitors of HAP formation. The amount of enzymatic hydrolysis of the polyphosphate has been reduced by the use of a linear molecularly dehydrated polyphosphate salt combined with fluoride as described in US-A-4,808,410.

Compounds containing a carbon atom covalently bonded to oxygen, the oxygen being covalently bonded to a phosphorous, herein referred to as C-O-P bonds, particularly six C-O-P bonds, such as phytic acid [myo-inositol 1,2,3,4,5,6-hexakis(dihydrogen phosphate)], have been recommended for various purposes in oral compositions. US-A-4,259,316 and US-A-4,335,102 disclose oral anticaries compositions containing a phytate compound and a stannous compound. Due to complex formation between polyvalent cations and phytate anion, the art teaches the presence of stannous compounds in an oral composition containing a phytate compound would not be desirable for inhibition of calculus formation.

In US-A-3,934,002 phytic acid is disclosed as one of the anticalculus compounds in oral compositions used together with a bisbiguanide antiplaque and anticaries agent. These two agents react with one another so that neither the anticalculus or antiplaque agent would be homogeneously distributed throughout the oral compositions. Since both agents are present, if a mouthrinse is prepared, it contains two visibly distinct phases, one being solid phase reaction product of bis-biguanide and anticalculus agent. US-A-4,263,276 and US-A-4,305,928 also describe visually clear oral compositions containing phytic acid in the presence of an alkali metal fluoride, monofluorophosphate or alkali metal monofluorophosphate, where a cationic material, such as a bisbiguanide or cationic surface active agent can be present. However, effective inhibition of HAP or plaque formation with compositions including compounds containing C-O-P bonds with an antimicrobial have not been known beyond such recommendations or speculation.

It would therefore be desirable to have an oral composition containing an effective antiplaque or anticalculus agent to aid in the prevention of dental caries and gingivitis as well as aid in the control of mouth malodor which does not stain the teeth and does not have a bitter taste. It would also be desirable to provide an antiplaque and anticalculus oral composition in which phytic acid is homogeneously distributed in the oral composition along with a cationic antimicrobial compound.

Furthermore, it would be desirable to provide an oral composition having enhanced retention of cationic antimicrobial compounds on the tooth surfaces useful in the prevention of dental plaque and gingivitis.

The present invention relates to oral compositions containing phytic acid or derivative thereof and a cationic antimicrobial compound and their use for the prevention of dental plaque. In particular, the present invention relates to an oral composition comprising: an orally acceptable vehicle containing:
(a) from 0.001 to 10 percent by weight of one or more compounds having C-O-P bonds selected from myo-inositol hexakis(dihydrogen phosphate), myo-inositol pentakis(dihydrogen phosphate), myo-inositol tetrakis(dihydrogen phosphate) and physiologically acceptable salts thereof;
(b) from 0.001 to 10 percent by weight of one or more cationic antimicrobial compounds;
(c) from 0.1 to 20 percent by weight of one or more compatibilizing agents; and
(d) an orally acceptable vehicle.

A further embodiment of the present invention provides an oral composition containing phytic acid or derivative thereof and a cationic antimicrobial compound which remain in solution, even in the presence of a polyvalent cation or polyvalent cations.

The present invention relates to an oral composition comprising an orally acceptable vehicle containing phytic acid or a said derivative thereof, a cationic component and a sufficient amount of a compatibilizing agent to inhibit the phytic acid or derivative thereof and an antimicrobial compound from interacting to form a precipitate when together in an aqueous solution. An "orally acceptable vehicle" means a medium in which an anticalculus or antiplaque agent may be administered to the oral cavity surfaces without substantial harmful effects to the surfaces thereof.

As plaque is a main etiological factor in gingivitis, periodontitis, tooth decay (dental caries) and other dental associated problems, the ability to control dental plaque aids in preventing and/or controlling gingivitis, periodontitis and dental caries. Thus, as used herein, "antiplaque" means antiplaque and/or antigingivitis and/or antiperiodontitis and/or anticaries. In addition, as the volatile sulfur compounds associated with oral malodor are related to the gingival health, as well as being produced by the putrefactive activity of microorganisms, as used herein, an antiplaque agent will also aid in the control of oral malodor.

To enhance the effect of preventing the formation of dental plaque, it has now been unexpectedly found that the retention of cationic antimicrobial compounds and physiologically acceptable salts thereof to a tooth surface can be substantially enhanced if the cationic antimicrobial compound is used in combination with phytic acid or a derivative thereof in the presence of a sufficient amount of a compatibilizing agent to prevent the phytic acid or derivative thereof and cationic antimicrobial compound from interacting to form a precipitate when exposed to each other in an aqueous environment. The ability of an antiplaque agent to remain in contact with the tooth surface to exert an anti-plaque effect is referred to as "substantivity" of the agent. It has also been unexpectedly found that in the presence of a compatibilizing agent, the phytic acid or derivative thereof and the cationic antimicrobial compound will remain in solution in the presence of polyvalent cations provided the ratio of polyvalent cation to phytic acid or derivative thereof is not greater than 5 to 1.

In certain preferred forms of the invention, the composition is substantially liquid in character, such as a mouthwash or rinse. In such a preparation the vehicle can be water or a water-alcohol mixture. When using a water-alcohol mixture, the weight ratio of water to alcohol is in the range of from 1:1 to 20:1, preferably 3:1 to 10:1 and more preferably 4:1 to 6:1. The total amount of water or water-alcohol mixture in this type of preparation is typically in the range of from 70 to 99.9 percent by weight of the preparation. The pH of such liquid, and other liquid preparations of the invention, is generally in the range of from 4.5 to 9, and typically from 5.5 to 8. The pH is preferably in the range of from 6 to 8. A preferred embodiment of the invention is as defined in Claim 6.

In certain other desirable forms of this invention, the oral composition may be substantially solid or semisolid in character, such as toothpowder, a dental tablet, a toothpaste, gel or dental cream. The vehicle of such solid or semisolid oral preparation generally contains added polishing material more fully described hereinafter.

As used herein, a "cationic antimicrobial compound" refers to an organic amine where the nitrogen is capable of being positively charged in an aqueous environment, and is represented by one or more of the following general formulae of A-J:
(A) Quaternary ammonium compounds represented by Formula I 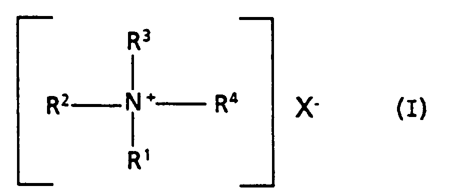 or Formula II 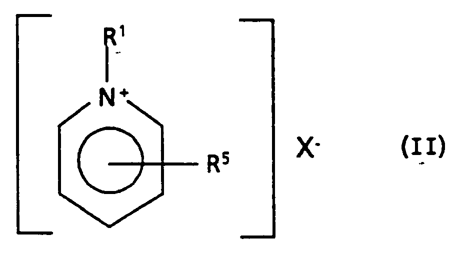 wherein:
   R¹ is a C₈-C₂₀ alkyl;
   R² is benzyl or C₁-C₁₂ alkyl;
   R³ and R⁴ are independently a C₁-C₇ alkyl or -(CH₂-CHOH-CH₂-O)ₙH wherein n is an integer from 1 to 6;
   R⁵ is -H, a C₁-C₇ alkyl or -(CH₂-CHOH-CH₂-O)ₙH wherein n is an integer from 1 to 6; and
   X⁻ is chloride (Cl⁻), bromide (Br⁻), iodide (I⁻) or fluoride (F⁻) ion;
(B) Pyridinium chlorides containing alkylthiomethyl or alkoxymethyl hydrophobic groups as disclosed by Weglowski et al., *J. Phar. Sci*., 80; 91-85 (1991), the disclosure of which is hereby incorporated by reference, having the formula 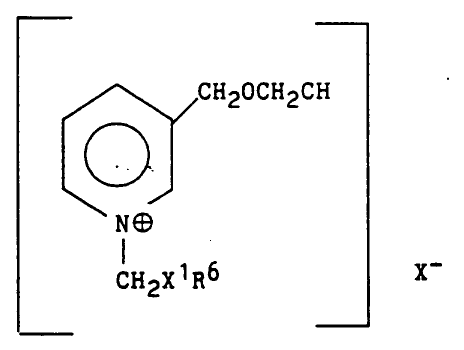 wherein
   X is as defined herein before and X¹ is oxygen or sulfur; and
   R⁶ is a C₄-C₁₆ alkyl or benzyl;
(C) Quaternary ammonium compounds that are esters of betaine and fatty alcohols, as disclosed by Linstedt et al., *Antimicrobial Agents and Chemotherapy*, 39, 1949-1954 (1990), the disclosure of which is hereby incorporated by reference, having the formula (CH₃)₃N^{⊕}-CH₂C(O)OR⁷ wherein R⁷ is a C₁₀-C₁₈ alkyl; and physiologically acceptable salts thereof;
(D) Sanguinarine and sanguinaria, sanguinaria being an extract from the bloodroot plant *Sanguinaria candensis*, the extract containing benzophenanthridine alkaloids such as sanguinarine, chelerythrine, protopine, homochelidonine and physiologically acceptable salts thereof as disclosed in US-A-4,145,412 and US-A-4,406,881, the disclosures of which are hereby incorporated by reference, sanguinaria being available in dentifrices under the trademark Viadent™ brand sanguinaria; the major active ingredient sanguinarine chloride salt having the formula 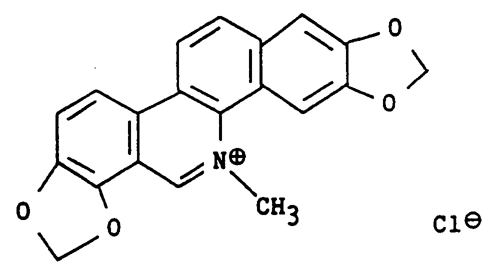
(E) Morpholine compounds as disclosed in US-A-4,894,221, the disclosure of which is hereby incorporate by reference, the morpholine compounds having the formula 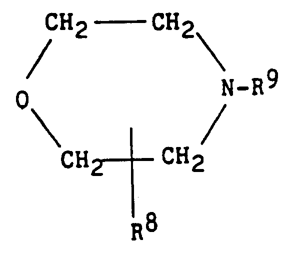 wherein
   R⁸ is a C₈-C₁₆ alkyl at the 2 or 3 position of the morpholino ring;
   R⁹ is a C₂-C₁₀ alkyl substituted with a hydroxy group at other than the alpha-position;
   the sum of R₈ and R₉ being greater than or equal to 10 and preferably 10-20; and physiologically acceptable salts thereof;
(F) Antibacterial secondary amines and amides as disclosed in *J. Antibacterial and Antifungal Agents*, 17, 371 (1989), the disclosure of which is hereby incorporated by reference, wherein the antibacterial compounds have the following formulae 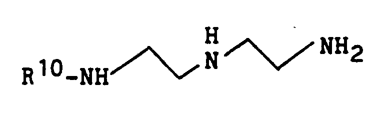 wherein R¹⁰ is a C₁₀-C₁₈ alkyl; 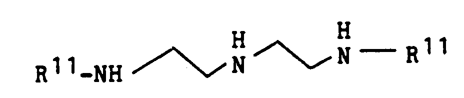 wherein each R¹¹ is independently C₈H₁₇ or C₁₀H₂₁; 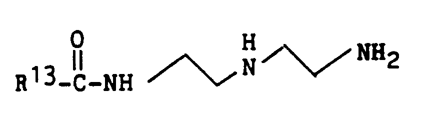 wherein R¹³ is a C₉-C₁₇ alkyl;
   or 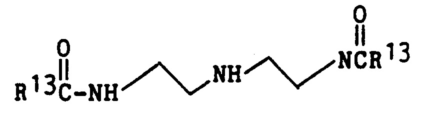 wherein each R¹³ is independently C₇H₁₅ or C₉H₁₉;
   and physiologically acceptable salts thereof;
(G) Dialkyl amines and N,N'-dialkylpolymethylenediamines as disclosed in *J. Antibacterial and Antifungal Agents*, 17, 579 (1989), the disclosure of which is hereby incorporated by reference, having the formula

   R¹⁴-NH-R¹⁴

   wherein each R¹⁴ is independently C₈H₁₇ or C₁₂H₂₅; or formula

   R¹⁵-NH(CH₂)ₙNH-R¹⁵

   wherein each R¹⁵ is independently a C₇-C₁₀ alkyl;
   n is an integer from 2 to 5; and physiologically acceptable salts thereof;
(H) N'-Alkyl-N-(2-aminoethyl)piperidine compounds as disclosed by Murata et al., *J. Pharm. Sci*., 80, 26-28 (1991), the disclosure of which is hereby incorporated by reference, the compounds having the formula 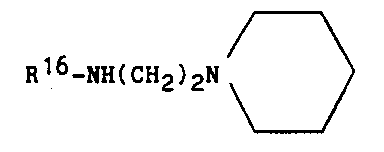 wherein R¹⁶ is a C₁₀-C₁₈ alkyl;
   and physiologically acceptable salts thereof;
(I) The ammonium compound 4-(2-propylenepentyl)-1-piperidinoethanol described in *J. Periodontal Research*, 18, pp. 429-437 (1983), the compound having the structure 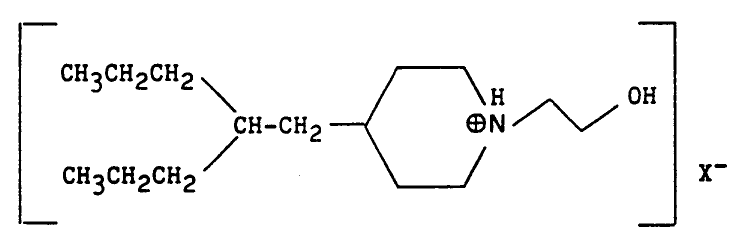 wherein X⁻ is as defined hereinbefore;
   described in the literature as Octapinal™ brand 4-(2-propylenepentyl)1-piperidinoethanol (Ferrosan AB, Sweden); and
(J) Alkyl-N-betaine in combination with an alkyl-N,N-dimethylamine oxide; the alkyl-N-betaine having the structure 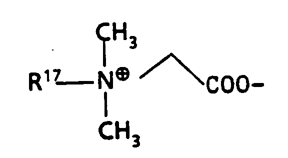 wherein R¹⁷ is a C₁₀-C₁₈ alkyl;
   the alkyl-N,N-dimethylamine oxide having the structure 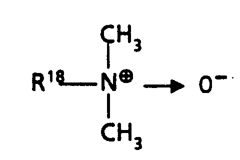 wherein R¹⁸ is a C₁₀-C₁₈ alkyl;
   as disclosed in US-A-4,839,158, the disclosure of which is hereby incorporated by reference.

As used herein, the term "alkyl" means a linear or branched alkyl and thus secondary and tertiary alkyls are included. The alkyl terms up to C₂₀ referred to herein include, for example, t-butyl, sec-butyl, isobutyl, and in like manner all such branched or straight chain alkyls.

Preferred quaternary ammonium antibacterial agents include dodecyl trimethyl ammonium bromide, benzyl dimethyl stearyl ammonium chloride, N-tetradecyl-4-ethylpyridinium chloride and cetylpyridinium chloride. The terms antibacterial and antimicrobial mean the ability to inhibit growth, metabolism or reproduction of microorganisms.

The cationic antimicrobial compounds useful in the present invention are commercially available or may be obtained by those of ordinary skill in the art without undue experimentation. For example, quaternary ammonium compounds may be produced by reacting alkyl halides with ammonia or primary amines, or by reacting a tertiary amine, pyridine or pyridine derivative with an alkyl halide. See, for example, Zoltewicz and Deady, *Adv. Heterocycl. Chem*., 22, 71-121 (1978); US-A-2,446,792; US-A-2,295,504 and US-A-4,994,199, the teachings of which are hereby incorporated by reference.

One or more cationic antimicrobial compounds are employed in amounts such that the oral product contains from 0.001 and 10 percent by weight of the antimicrobial compound. Preferably for desired levels of antiplaque and antigingivitis effect, the finished oral product contains 0.01 to 5 percent and preferably 0.025 to 1.0 percent by weight of the antimicrobial compound. Typically a singular antimicrobial compound is employed in the oral product.

The compounds of the present invention which contain C-O-P bonds are phosphate esters of myo-inositol, such as phytic acid, also known as myo-inositol hexakis(dihydrogen phosphate), inositol hexaphosphoric acid, and 1,2,3,4,5,6-cyclohexanehexanol-phosphoric acid. As used herein "phytic acid compound" means the hexakis phosphate ester of myo-inositol, myo-inositol hexakis(dihydrogen phosphate), and the lesser substituted tetrakis and pentakis phosphate esters of myo-inositol, myo-inositol tetrakis(dihydrogen phosphate) and myo-inositol pentakis (dihydrogen phosphate) respectively, and physiologically acceptable salts thereof, such as alkali metal, alkaline-earth metal, ammonium salts or mixtures thereof. These phytic acid compounds may be used singly or in combination. Suitably, phytin, which is the calcium magnesium salt of phytic acid represented by the formula Ca₅Mg(C₆H₁₂O₂₄P₆.3H₂O)₂, is used in the present invention in addition to or replacement of phytic acid.

Phytic acid and phytin are commercially available. The tetrakis and pentakis phosphate esters of inositol compounds can be prepared by hydrolyzing sodium phytate with hydrochloric acid and separating the inositol phosphates by high performance liquid chromatography as described by Sanberg and Ahderinne, J. Food Sci., 51, 547-550 (1986), the disclosure of which is hereby incorporated by reference.

Phytic acid compound is present in the oral composition of the present invention, in an amount from 0.001 to 10 percent by weight. When the oral composition is essentially liquid in nature, the phytic acid compound is preferably present in an amount from 0.001 to 10 percent by weight, preferably from 0.005 to 5 percent and more preferably from 0.01 to 1 percent by weight.

When the oral composition is essentially liquid in nature, to maintain the cationic antimicrobial compound and phytic acid compound in solution, it is desirable that the composition contain a sufficient amount of a compatibilizing agent to keep the phytic acid compound and cationic antimicrobial compound from interacting to form a precipitate. Compatibilizing agents in the present invention are those which do not have a detrimental effect on the substantivity of the cationic antimicrobial compound in the presence of phytic acid compound and maintains the cationic antimicrobial compound and phytic acid compound in solution when the oral composition is essentially liquid in nature such that an aqueous solution of the phytic acid compound, cationic antimicrobial compound and compatibilizing agent does not visually become turbid after standing for 30 minutes at room temperature. A detrimental effect on substantivity means the retention of the antimicrobial compound near the tooth surface in the presence of phytic acid compound is not substantially different than the retention of the antimicrobial compound in the absence of phytic acid compound. It is therefore possible that a compound or combination of compounds may combatibilize the phytic acid compound and antimicrobial compound, i.e., keep them in solution, but adversely affect the substantivity of the antimicrobial/phytic acid compound solution.

When the oral composition is substantially gel-like or semisolid in form, the vehicle of such solid oral preparation contains a liquid moiety of water so that the phytic acid compound, cationic antimicrobial compound and compatibilizing agent are homogeneously distributed throughout the liquid phase of the composition. The total amount of water in gel-like or semisolid-like oral compositions is typically in the range of from 5 to 60 percent by weight of the preparation, preferably from 10 to 50 percent.

While not wishing to be bound by theory, it is believed the compatibilizing agents of the present invention reduce the interaction between the phytic acid compound and cationic antimicrobial compound, reducing or preventing the formation of a precipitate when these two compounds are exposed to each other in an aqueous environment. The amount of compatibilizing agent in the oral compositions of the present invention is from 0.1 to 20 percent by weight, preferably from 0.1 to 10 percent by weight of the total composition. Particularly useful compatibilizing agents for oral compositions of the present invention which are substantially liquid in nature are acids and their alkali metal or alkaline-earth metal salts, or mixtures thereof. The mixtures are designated herein as anionic buffers. Suitable anionic buffers are, for example, phosphate, acetate, borate, citrate, bicarbonate, gluconate, tartrate, sulfate, and the like, or mixtures thereof. The preferred anionic buffers being phosphate and bicarbonate. When the oral composition is essentially in the liquid form, the anionic buffer is present in a concentration of from 0.1 M to 1.0 M, preferably from 0.25 M to 0.75 M.

Other examples of compatibilizing agents useful in the present invention are surfactants which maintain the phytic acid compound and antimicrobial compound in solution and does not interfere with enhanced substantivity due to the presence of phytic acid compound. An example of a particularly suitable nonionic surfactant is poly(oxyethylene), poly(oxypropylene) block polymers known as poloxamers and available, for example, under the trademark "PLURONICS" (BASF Wyandotte Co., Parsippany, NJ). Another example of a particularly suitable nonionic surfactant is polyethylene oxide sorbitan esters, available for example, under the trademark "TWEENS" (ICI American Inc., Wilmington,De.) Suitable anionic surfactants include, for example, anionic surfactants produced from fatty acids and the amino acid sarcosine, such as N-lauroyl sarcosine, available for example, under the trademark "HAMPOSYL" by W.R. Grace and Co. (CT).

When utilizing a combination of compatibilizing agents, it is desirable that the total amount of compatibilizing agent in the oral composition remain from 0.1 to 20 weight percent. The concentration of compatibilizing agent or agents for use in the present invention can be readily determined by those of ordinary skill in the art based upon the teachings herein.

In a further embodiment, it has been found that when a polyethylene oxide sorbitan ester is used as a compatibilizing agent for phytic acid compound and an antimicrobial compound such as N-tetradecyl-4-ethylpyridinium bromide, the concentration of compatibilizing agent can be reduced below 0.1 weight percent, and about 0.05 weight percent can be used.

The molar ratio of phytic acid compound to the cationic antimicrobial compound in the presence of a compatibilizing agent is preferably from 10:1 to 1:10, more preferably from 5:1 to 1:5, and most preferably about 1:1.

Preparation of the oral compositions of the present invention can be made by using customary procedures for unifying components applied to the teeth and gingiva. It has been found that liquid mouthwashes and topical solutions of the present invention, can be prepared by: (a) dissolving the phytic acid compound and compatibilizing agent in water, (b) adjusting the pH to between 6 to 8, and then (c) adding the cationic antimicrobial compound. When the oral compositions of the present invention contain a polyvalent metal ion in addition to the phytic acid compound, the compatibilizing agent and the cationic antimicrobial compound, then the compositions are advantageously prepared by: (a) dissolving the metal ion, phytic acid compound, and a compatibilizing agent in water, (b) adjusting the pH to between 6 to 8, and then (c) adding the cationic antimicrobial compound. Alternatively, the cationic antimicrobial compound can be added to the solutions above, prior to adjusting the pH. Other components, such as sweetening and flavoring agents as described more fully herein, can then be added if desired.

To prepare an oral composition which is substantially solid or semisolid in character, an aqueous solution of the phytic acid compound, cationic antimicrobial compound and compatibilizing agent is prepared in the ratios as described above, and the water removed. Alternatively, a substantially solid or semisolid oral composition containing phytic acid compound, cationic antimicrobial compound and compatibilizing agent may be prepared by mixing the components in the preferred ratios with the other ingredients of the oral composition as described herein.

It has been unexpectedly found that a metal ion selected from copper, strontium (Sr²⁺), magnesium (Mg²⁺), tin (Sn²⁺), zinc (Zn²⁺), calcium (Ca²⁺) or mixtures thereof, can be added to the oral composition containing the phytic acid compound, cationic antimicrobial compound and compatibilizing agent without the phytic acid compound precipitating from solution. The molar ratio of the metal ion to the phytic acid compound which can be present in the oral compositions of the present invention is from 4:1 to 1:4, preferably from 3:1 to 1:3, and more preferably about 1:1. The inclusion of a metal ion with the phytic acid compound and antimicrobial agent would aid in the suppression of oral malodor in addition to aiding in the control of calculus, plaque and gingivitis due to the inhibitory effect of the antimicrobial compound.

The dentifrices of the present invention may also be in a kit form for treating the oral cavity, the kit comprising phytic acid compound in an orally acceptable vehicle, one or more compatibilizing agents in an orally acceptable vehicle, and one or more cationic antimicrobial compounds in an orally acceptable vehicle; and a means to contain the phytic acid compound separately from the cationic antimicrobial. Means to separate the phytic acid compound and cationic antimicrobial includes placing them in separate vessels or in a compartmentalized container. The compatibilizing agent may be mixed with the phytic acid compound, with the cationic antimicrobial or may be contained separately.

When the dentifrice of the present invention is in a kit form, the phytic acid compound, compatibilizing agent and cationic antimicrobial compound is mixed prior to application.

When mixing the phytic acid compound, compatibilizing agent and cationic antimicrobial compound prior to application to the oral cavity, it may be necessary to increase their concentration to account for dilution effects which can occur upon mixing. When applying the phytic acid compound, compatibilizing agent and cationic antimicrobial in a kit form by mixing prior to use, the concentration of the individual compounds to which the oral cavity is exposed should be in the range given hereinbefore for their concentration in the final dentifrice product.

A variety of other ingredients may be added to the dentifrices of the present invention. Thus for example, prophylactic agents, polishing agents, soaps or detergents, flavoring and sweetening agents, thickening agents and humectants may be included using techniques which are known in the art.

Representative prophylactic agents include supplemental caries-preventing materials such as sodium fluoride, stannous fluoride, potassium fluoride, hexylamine hydrofluoride, myristylamine hydrofluoride, betaine fluoride, glycine potassium fluoride, etc. A particularly preferred fluoride is sodium fluoride. Typically these prophylactic agents are present in sufficient concentrations so as to provide an available fluoride ion concentration of up to 2 percent by weight, and preferably in the range of from 0.5 to 2 percent by weight, of the dentifrice composition.

Suitable polishing agents include, for example, abrasive materials such as insoluble condensed phosphates such as calcium pyrophosphate, insoluble calcium polyphosphate (also known as calcium polymetaphosphate) and highly polymerized sodium polyphosphate; and water impervious cross-linked thermosetting resins such as the condensations products of melamine and urea with formaldehyde. Other suitable polishing agents will be obvious to those skilled in the art.

The polishing material is generally present in the solid or semisolid compositions in weight concentrations of from 10 to 99 percent. Preferably, it is present in amounts ranging from 20 to 75 percent in toothpaste, and from 70 to 99 percent in tooth powder.

Soaps or detergents may also be employed in the present invention to lower the surface tension to achieve increased prophylactic action, assist in achieving thorough and complete dispersion of the anti-calculus agent and render the instant compositions more cosmetically acceptable. Suitable soaps include, for example, the soaps of high molecular weight fatty acids such as sodium and potassium soaps of myristic, stearic or palmitic acids and fatty acids mixtures of palm oil and coconut oil. Typical synthetic detergents include alkyl sulfates and sulfonates having alkyl groups of from 8 to 18 carbon atoms, such as, for example, sodium lauryl sulfate, the sulfated fatty alcohols derived from coconut oil and palm oil. The soaps typically comprise up to 5 percent by weight of the dentifrice composition.

Any suitable flavoring or sweetening material may also be employed. Examples of suitable flavoring constituents are flavoring oils, e.g., oil of spearmint, peppermint, wintergreen, sassafras, clove, sage, eucalyptus, marjoram, cinnamon, lemon and orange and methyl salicylate. Suitable sweetening agents include sucrose, lactose, maltose, sorbitol, xylitol, sodium cyclamate, perillartine, APM (aspartylphenylalanine, methyl ester), saccharine and the like. Suitably, flavor and sweetening agents may together comprise from 0.1 percent to 5 percent of the preparation.

Toothpastes, creams and gels typically contain a natural or synthetic thickener or gelling agent in proportions of from 0.1 to 10 percent, preferably from 0.5 to 5 percent, by weight. Suitable gelling or thickening agents include for example, water-soluble salts of cellulose ethers such as sodium carboxymethyl cellulose and sodium carboxymethylhydroxyethyl cellulose; natural gums such as gum karaya, gum arabic, and gum tragacanth; and colloidal magnesium aluminum silicate or finely divided silica.

Suitable humectants which may be employed in compositions of the invention include glycerine, propylene glycol, sorbitol, polypropylene glycol and/or polyethylene glycol and other polyhydric alcohols. The humectants may comprise from 10 to 90 percent by weight of the dentifrice composition.

The invention will be further clarified by a consideration of the following examples, which are intended to be purely exemplary of the present invention.

### GENERAL EXPERIMENTAL

### Preparation of Stock Solutions

0.045 M phytic acid. A 0.045 molar (M) solution of phytic acid was prepared by dissolving 1.462 g (1.125 millimoles (mMol)) of 50 percent by weight of phytic acid (Jonas Chemical Corp.) in 10 ml of water. The pH of this solution was brought to 7.78 by the addition of 1 N NaOH. This solution was transferred quantitatively to a 25 ml volumetric flask and diluted to the mark with water.
0.0045 M phytic acid. A 0.0045 M solution of phytic acid was prepared by adding 0.743 ml of phytic acid (40 percent weight solution, Aldrich Chemical Co., Inc.) to a 100 ml volumetric flask and diluting to the mark with water.
0.0045 M CPC. A 0.0045 M solution of cetylpyridinium chloride (CPC) was prepared by adding 0.4026 ± 0.0001 g of CPC (Aldrich Chemical Co., Inc.) to a 250 ml volumetric flask, dissolving in water and diluting to mark with water. The final pH of the solution was 7.4.
0.045 M HEDP. A 0.045 M solution of hydroxyethylidenediphosphonic acid (HEDP) was prepared by adding 0.0618 ± 0.0001 g of 60 percent active HEDP (MAYO Chemical Co.) to a 60 ml beaker and adding 40 ml of water. This solution was adjusted to pH 7.6 using a few drops of 1.0 N sodium hydroxide.
1.5 M phosphoric acid. A 1.5 M phosphoric acid solution was prepared by adding 25 ml of water to a beaker containing 17.29 g of 85 percent by weight solution of phosphoric acid (Mallinckrodt). The pH of this solution was raised to about 7 with the addition of 50 percent by weight sodium hydroxide. The solution was then transferred quantitatively to a 100 ml volumetric flask and diluted to the mark with water.

### Glycolysis pH Test

A sucrose solution was prepared by loading 1.0 g of sucrose (Imperial Pure Cane Sugar) into a 60 ml beaker and then adding 20 ml of water. To this solution was added 8.0 ml of pooled whole human saliva. The saliva was collected from donors who had been permitted to eat or drink anything prior to collection period, but had foregone any oral hygiene on the day of collection. Prior to the collection, each donor rinsed their mouth for thirty seconds with approximately 30 ml water, and after waiting about 5 minutes, began collecting saliva for 30 to 40 minutes, keeping the collected saliva on ice.

To the saliva/sucrose solution was added 1.0 ml of brain/heart infusion broth containing *Streptococcus mutants* (American Type Culture Collections No. 25175, ATCC) and 1.0 ml of brain/heart infusion broth containing *Streptococcus sanguis* (ATCC #10556). These cultures had been inoculated into 40 ml of broth and grown at 37°C for sixteen hours prior to adding to the saliva/sucrose solution. (Each broth contained approximately 60 million colony forming units at the time of addition.)

Aliquots of 0.75 ml of the above saliva/sucrose/bacterial solution were added to the test tubes containing various washed HAP suspensions. These test tubes were capped and attached to a tube rotator and placed in a 37°C incubator for sixteen hours. Following this incubation period, the rotator was removed from the oven and allowed to cool to ambient temperature. The pH of the solutions were checked with a pH meter using a pH electrode calibrated with pH 4, 7 and 10 buffers.

### Treating and Washing Hydroxyapatite

To determine the substantivity of CPC in combination with phytic acid, the following washing procedure of the hydroxyapatite was done prior to performing a glycolysis pH test:
A 60 ml beaker was loaded with 12.0 g of hydroxyapatite (HAP) in a buffer suspension (25 percent by weight solids from Sigma Chemical Co.) and washed with 25 ml of water. The HAP suspension was filtered through a medium glass fitted filter to obtain a HAP filter cake. The HAP filter cake was washed a second time with an additional 25 ml of water and then filtered through a glass filter funnel. The white solid filter cake containing 3.0 g of HAP without the buffer was resuspended with 30.0 ml of water to produce a 3.0 g HAP/30.0 ml or 100 mg/ml suspension.

Two ml of the HAP suspension was transferred to each of several sterile-disposable polystyrene 5 ml test tubes labeled D₁--Dₙ (where n = number of test solutions). Two ml of a test solution were then added to each test tube.

The tubes containing the HAP and test solution were capped and attached to a tube rotator and rotated end-over-end to allow the test solutions to contact the HAP for a total of ten minutes.

After mixing, the test tubes were placed in an Industrial Equipment Company (IEC) model K centrifuge and spun at setting 25 (mid-range) for ten minutes. The tubes were removed and the liquid layer decanted. A macro-pipettor was then used to add 3.0 ml of water to each test tube containing the centrifuged hydroxyapatite. The HAP solids were resuspended by vigorous in-and-out flowing action through the pipette. The tubes were again centrifuged at setting 25 for ten minutes and the liquid layer decanted. Following the three milliliter wash step, the HAP solids were resuspended in 2.0 ml of water to produce the original 100 mg/ml suspension concentration. A 0.5 ml sample (containing 50 mg HAP) of this HAP suspension was removed and placed in each of several 5 ml polystyrene test tube labeled A₁--Aₙ. This sample A contains one fourth of the original HAP suspension which has been washed with three milliliters of water.

The remaining 1.5 ml in test tubes labeled D₁--Dₙ were centrifuged for ten minutes, the test tubes removed, and the liquid layer decanted. Three milliliters of water were added to these test tubes and the HAP solids resuspended/washed using disposable pipettes. The tubes were centrifuged for ten minutes, the tubes removed and the liquid layer decanted.

An additional three milliliters of water were added to these tubes and the HAP solids resuspended/washed by pipette. These tubes were again placed in the centrifuge and spun for ten minutes. The tubes were removed, the liquid layer decanted and 1.5 ml of water added to each tube. The HAP solids were resuspended to the original 100 mg/ml concentration and a 0.5 ml sample removed and placed in each of several 5 ml polystyrene test tubes labeled B₁--Bₙ. This sample B contained 50 mg HAP solids which had been treated with test solution and then washed with a total of eleven milliliters of water.

The procedure given above was repeated a third and fourth time to create a series of test tubes labeled C₁--Cₙ and D₁--Dₙ. The C samples contained HAP solids which had been treated with test solution and then washed with a total of 18.5 ml of water. The D samples contained HAP solids which had been treated with the test solution and then washed with a total of 25.5 ml water.

A glycolysis pH test was then performed as described above by adding 0.75 aliquots of the saliva/sucrose/bacterial mixture to the test tubes labeled A₁--Dₙ, each containing 0.5 ml of the treated washed HAP suspension.

### Example 1: Incompatibility of CPC with phytic acid

Into each of eight vials was placed 500 µl of 0.0045 M CPC, varying amounts of a 0.045 M phytic acid solution and water as shown in Table I to give a final concentration of 1.5 mM CPC in all the samples. The phytic acid solution and water were combined first.

The vials were then capped and allowed to mix on an end-over-end rotator for 18 hours to allow for complete precipitation. At the end of this time, the tubes were centrifuged at 4,000 rpm for 5 minutes and a 200 µl aliquot of the supernatant removed and added to three ml of water. These diluted samples were then mixed in a quartz cuvette and the ultraviolet (UV) absorption determined at 260 nanometers. The amount of CPC still in solution was determined by comparing the absorbence of the samples to the absorbence of a 0.0015 M CPC solution. The results, as shown in Table I, show CPC precipitates in the presence of phytic acid.

**TABLE I**

| µl Phytic Acid | µl Water | mM CPC* | mM Phytic Acid | % of CPC* still in Solution |
|---|---|---|---|---|
| 1000 | 0 | 1.5 | 30 | 21.3 |
| 500 | 500 | 1.5 | 15 | 10.7 |
| 100 | 900 | 1.5 | 3 | 3.5 |
| 50 | 950 | 1.5 | 1.5 | 1.9 |
| 33.3 | 966.7 | 1.5 | 1.0 | 1.8 |
| 26.7 | 973.7 | 1.5 | 0.8 | 1 |
| 10 | 990 | 1.5 | 0.3 | 2.9 |
| 5 | 995 | 1.5 | 0.15 | 13.4 |

| | | | | |
|---|---|---|---|---|
| *CPC = cetylpyridinium chloride | | | | |

### Example 2

The procedure in Example 1 was repeated except the CPC and phytic acid solutions were made in 0.5 M phosphate buffer at pH 7.3. The phosphate buffer was prepared from 85 percent by weight of phosphoric acid and adjusted to pH about 7.3 with 50 percent by weight of sodium hydroxide. The phosphate buffer was also substituted for the water portion of the solutions. The percent of CPC remaining in solution was determined by measuring the ultraviolet absorbence of the samples compared to a 0.0015 M CPC standard. The results, given in Table II, show no CPC is precipitated from solution at any of the various concentrations and demonstrate the compatibilization of CPC with phytic acid in the presence of an anionic buffer.

**TABLE II**

| µl Phytic Acid | µl Phosphate Buffer | mM Phytic Acid | % of CPC* still in Solution |
|---|---|---|---|
| 1000 | 0 | 30 | 105.8 |
| 500 | 500 | 15 | 102.4 |
| 100 | 900 | 3 | 103.2 |
| 50 | 950 | 1.5 | 103.6 |
| 33.3 | 966.7 | 1.0 | 103 |
| 26.7 | 973.7 | 0.8 | 100.4 |
| 10 | 990 | 0.3 | 103.7 |
| 5 | 995 | 0.15 | 101.7 |

| | | | |
|---|---|---|---|
| *CPC = cetylpyridinium chloride | | | |

### Example 3

Using the experimental procedures described above for glycolysis test and preparation of the hydroxyapatite, the following compounds were tested for HAP substantivity: water (control); cetylpyridinium chloride (CPC); phytin/phosphoric acid/citric acid (PyPCi); hydroxyethylidene phosphate (HEDP); hydroxyethylidene phosphate/cetylpyridinium chloride (HEDP/CPC); and phytin/phosphoric acid/citric acid/cetylpyridinium chloride (PyPCiC). All the compounds tested were 0.0015 M except for phosphate and citrate which were 0.015 M.

The 0.0015 M CPC and 0.0015 M HEDP solutions were prepared by diluting 0.0045 M stock solutions prepared as previously described.

The PyPCi (phytin/phosphate/citrate) solution was prepared by placing 123 mg (75 µmoles) of phytin (American Tokyo Kasei, Inc.) and 4 ml of 0.15 M citric acid into a 4 ounce (120 ml) jar. (The citric acid being prepared by adding 1.575 g of citric acid to water and bringing the final volume to 50 ml in a volumetric flask). To this suspension of phytin and citric acid was added 2.873 g (0.025 mole) of neat phosphoric acid followed by 30 ml of water. The pH was adjusted to 7.38 by the addition of 50 percent by weight of sodium hydroxide. After allowing the solution to cool to room temperature, an additional 10 ml of water was added to bring the volume to 50 ml. The pH was 7.36. The solution had a faint turbidity and was centrifuged and the supernatant decanted giving a clear solution.

The PyPCiC (phytin/phosphate/citrate/cetyl-pyridinium chloride) solution was prepared by placing 5.37 mg of CPC into a 10 ml volumetric flask and bringing to the 10 ml mark using the phytin/phosphate/citrate solution described above.

The HEDP/CPC (hydroxyethylidene phosphate/cetyl-pyridinium chloride solution) was prepared by placing 20 ml of a 0.0045 M HEDP solution into a 4 ounce (120 ml) jar and adding 20 ml of water and 20 ml of a 0.0045 M CPC stock solution. The pH was adjusted to 7.4 with the dropwise addition of 1.0 N sodium hydroxide.

The phosphate buffer/CPC (Phos/CPC) solution was prepared by adding 20 ml of a 0.042 M sodium phosphate buffer solution to a four ounce (120 ml) jar and diluting with 20 ml of water and 20 ml of a 0.0045 M CPC stock solution. The 0.042 M sodium phosphate buffer was prepared by adding 0.2318 ± 0.0001 g of NaH2PO4.H₂O (J.T. Baker Chemical Co.) to a 60 ml beaker and adding 40 ml of water and adjusting the pH to 7.36 by the dropwise addition of 1 N sodium hydroxide.

The results of the glycolysis pH test for HAP treated by the above compounds are shown Table III.

**TABLE III**

| pH AS A FUNCTION OF HAP WASHINGS | | | | | | | |
|---|---|---|---|---|---|---|---|
| Volume of Wash (ml) | Water | CPC¹ | PyPCi² | HEDP³ | HEDP/CPC⁴ | Phos/CPC⁵ | PyPCiC⁶ |
| 3 | 5.04 | 6.98 | 5.07 | 5.04 | 7.04 | 7.02 | 7.3 |
| 11 | 5.12 | 5.29 | 5.12 | 5.5 | 5.87 | 5.84 | 7.01 |
| 18.5 | 5.06 | 5.13 | 5.18 | 5.21 | 5.3 | 5.18 | 7.04 |
| 25.5 | 5.14 | 5.35 | 5.2 | 5.15 | 5.29 | 5.1 | 7.04 |

| | | | | | | | |
|---|---|---|---|---|---|---|---|
| ¹CPC = cetylpyridinium chloride; | | | | | | | |
| ²PyPCi = phytin, phosphate and citrate; | | | | | | | |
| ³HEDP = hydroxyethylidene phosphate; | | | | | | | |
| ⁴HEDP/CPC = hydroxyethylidene phosphate and cetylpyridinium chloride; | | | | | | | |
| ⁵Phos/CPC = phosphate and cetylpyridinium chloride; | | | | | | | |
| ⁶PyPCiC = phytin, phosphate, citrate and cetylpyridinium chloride; | | | | | | | |

These results show that retention of cetylpyridinium chloride as measured by the retention of antimicrobial activity was greatest for the PyPCiC solution.

### Example 4

Using the experimental procedures described above for the glycolysis test and preparation of the hydroxyapatite, the following compounds were tested for HAP substantivity: water (control); cetylpyridinium chloride (CPC); phytin/phosphoric acid/citric acid/cetylpyridinium chloride (PyPCiC); and phytic acid/phosphoric acid/citric acid/cetylpyridinium chloride (PaPCiC). The CPC and PyPCiC solutions were prepared as given in Example 3. The phytic acid/phosphoric acid/citric acid/cetylpyridinium chloride (PaPCiC) solution was prepared by adding 77.2 µl (150 µmoles) of phytic acid (40 percent by weight, Aldrich Chemical Co., Inc.), 2.873 g of phosphoric acid, 4 ml of 0.15 M citric acid and 25 ml water to a vial. This solution was brought to pH 7.39 by the addition of 3.71 g of 50 percent by weight of sodium hydroxide and diluted to mark with water in a 50 ml volumetric flask. A 5.37 mg portion of CPC was dissolved in 10 ml of this solution to produce a solution containing 3 mM phytic acid, 12 mM citrate, 1.5 mM CPC and 500 mM phosphate.

The results of the glycolysis pH test for HAP treated by the above compounds are shown in Table IV.

**TABLE IV**

| pH as a Function of HAP Washings | | | | |
|---|---|---|---|---|
| Volume of Wash (ml) | Water | CPC¹ | PyPCiC² | PyPCiC³ |
| 3 | 4.83 | 7.12 | 7.34 | 7.40 |
| 11 | 4.91 | 6.02 | 7.07 | 7.14 |
| 18.5 | 4.93 | 5.15 | 7.18 | 7.22 |
| 25.5 | 5.17 | 5.35 | 7.17 | 7.24 |

| | | | | |
|---|---|---|---|---|
| ¹CPC = cetylpyridinium chloride | | | | |
| ²PyPCiC = phytin, phosphate, citrate and cetylpyridinium chloride | | | | |
| ³PaPCiC = phytic acid, phosphate, citrate and cetylpyridinium chloride | | | | |

These results show that both phytin and phytic acid enhance the substantivity of CPC to hydroxyapatite as measured by the gylcolysis pH test.

### Example 5

Preparation of the HAP and the treatment of the HAP with test solutions was as described under general experimental. However, the amount of water wash of the HAP suspensions was increased from three to 10 ml. As a result, samples A, B, C and D were removed after washing with 10, 31, 51.5 and 71.5 ml of water, respectively. Using this wash procedure, the solutions as prepared in Example 4 were tested for HAP substantivity: water (control); cetylpyridinium chloride (CPC); phytin/phosphoric acid/citric acid/cetylpyridinium chloride (PyPCiC); phytic acid/phosphoric acid/citric acid/cetylpyridinium (PaPCiC).

The results of the glycolysis pH test for HAP treated by the above compounds are shown in Table V.

**TABLE V**

| pH Drop as a Function of HAP Washings | | | | |
|---|---|---|---|---|
| ml of Water Wash | Water | CPC¹ | PyPCiC² | PyPCiC³ |
| 10 | 4.76 | 7.15 | 7.32 | 7.26 |
| 31 | 4.83 | 5.04 | 7.24 | 7.21 |
| 51.5 | 4.88 | 5.01 | 7.34 | 7.30 |
| 71.5 | 5.19 | 5.17 | 7.36 | 7.28 |

| | | | | |
|---|---|---|---|---|
| ¹CPC = cetylpyridinium chloride | | | | |
| ²PyPCiC = phytin, phosphate, citrate and cetylpyridinium chloride | | | | |
| ³PaPCiC = phytic acid, phosphate, citrate and cetylpyridinium chloride | | | | |

The results show that retention of cetylpyridinium chloride as measured by the retention of antimicrobial activity was substantially enhanced by the presence of phytic acid or phytin and an anionic buffer under extended washing procedures.

### Example 6

Using the wash procedure of 10, 31, 51.5 and 71.5 ml of Example 5, the following compounds were tested for HAP substantivity: water (control); cetylpyridinium chloride (CPC); phytic acid/phosphoric acid/cetylpyridinium chloride (PaPC). The CPC was 0.0015M as described in Example 3. The PaPC solution was prepared by adding 92.6 µl of phytic acid (40 percent weight solution, Aldrich Chemical Co., Inc.) and 20 ml of water to a 100 ml beaker. To this solution was added 3.45 g of phosphoric acid (85 percent by weight solution, Mallinckrodt) and the pH adjusted to 7.4 with the dropwise addition of 0.85 g of 50 percent by weight sodium hydroxide solution. The volume of the solution was then brought to 40 ml. This phytic acid/phosphate buffer solution was then formulated with 20 ml of 0.0045 M CPC stock solution to produce a formulation containing 0.0012 M phytic acid, 0.5 M phosphate buffer, 0.0015 M cetylpyridinium chloride. The results of the glycolysis pH test for HAP treated by the above compounds are shown in Table VI.

**TABLE VI**

| pH as a Function of HAP Washings | | | |
|---|---|---|---|
| ml of Water Wash | Water | CPC¹ | PaPC² |
| 10 | 5.29 | 7.38 | 7.59 |
| 31 | 5.29 | 5.55 | 7.49 |
| 51.5 | 5.29 | 5.43 | 7.41 |
| 71.5 | 5.21 | 5.33 | 7.54 |

| | | | |
|---|---|---|---|
| ¹CPC = cetylpyridinium chloride | | | |
| ²PaCP = phytic acid, phosphate and cetylpyridium chloride | | | |

The results show that retention of cetylpyridinium chloride as measured by the retention of antimicrobial activity is substantially enhanced during extended water washing by the presence of phytic acid with only phosphate buffer to compatibilize the phytic acid and cetylpyridinium chloride.

### Example 7

The preparation of the HAP and the treatment of the HAP with test solutions was as previously described except in addition to using a 10 ml wash, the number of washing steps was doubled. Thus samples A, B, C and D are thus removed after washing with 22, 63.5, 84.5 and 105 ml respectively. Using this procedure, the following compounds were tested for HAP substantivity: water (control); cetylpyridinium chloride (CPC); and the following metals with phytic acid/phosphate/cetyl-pyridinium chloride: calcium: (Ca-PaPC); magnesium (Mg-PaPC); tin (Sn-PaPC); zinc (Zn-PaPC); strontium (Sr-PaPC); and copper (Cu-PaPC). The CPC was prepared as in Example 3. The formulations containing the metals were prepared by weighing into labeled jars the amount of metal chloride as given in Table VII.

**TABLE VII**

| Preparation of Metal Chloride Solutions | | | |
|---|---|---|---|
| Metal | Formula | Wt. (g) added to jar | FW* |
| Calcium | CaCl₂·H₂O | 0.0298 | 147.02 |
| Zinc | ZnCl₂ | 0.0277 | 136.28 |
| Strontium | SrCl₂·6H₂O | 0.0540 | 266.62 |
| Tin | Sn(II)Cl₂·2H₂O | 0.0457 | 225.63 |
| Magnesium | MgCl₂·6H₂O | 0.0411 | 203.31 |
| Copper | CuCl₂ | 0.0273 | 134.45 |

| | | | |
|---|---|---|---|
| *FW = formula weight | | | |

A 15 ml aliquot of a 0.0045 M phytic acid solution and a 15 ml aliquot of a 1.5 M phosphoric acid solution were then added to each jar and the pH adjusted to about 7 with the dropwise addition of 50 percent sodium hydroxide. A 15 ml aliquot of a 0.0045 M CPC solution was then added to each of the above solutions to produce formulations containing 0.0015 M metal; 0.0015 M phytic acid; 0.5 M phosphate; and 0.0015 M CPC. The solutions containing zinc, strontium or copper were initially water clear, with a precipitate observed after several hours.

The HAP substantivity of the formulations containing the metal as measured by the glycolysis pH test are given in Table VIII.

**TABLE VIII**

| pH as a Function of HAP Washings¹ | | | | | | | | |
|---|---|---|---|---|---|---|---|---|
| Volume of Wash (ml) | Water | CPC | Ca-PaPC | Mg-PaPC | Sn-PaPC | Zn-PaPC | Sr-PaPc | Cu-PaPC |
| 22 | 5.12 | 5.92 | 7.59 | 7.56 | 7.55 | 7.61 | 7.62 | 7.61 |
| 63.5 | 5.22 | 5.24 | 7.67 | 7.66 | 7.64 | 7.71 | 7.72 | 7.66 |
| 84.5 | 5.33 | 5.34 | 7.75 | 7.77 | 7.69 | 7.78 | 7.81 | 7.73 |
| 105 | 5.43 | 5.44 | 7.76 | 7.80 | 7.73 | 7.82 | 7.84 | 7.76 |

| | | | | | | | | |
|---|---|---|---|---|---|---|---|---|
| ¹CPC = cetylpyridinium chloride; PaPC = phytic acid, phosphate and cetylpyridinium chloride; Ca = calcium; Mg = magnesium; Sn = tin; Zn = zinc; Sr = strontium; Cu = copper(II) | | | | | | | | |

These results show that the presence of metal ions does not interfere with the ability of phytic acid to enhance the substantivity of CPC to hydroxyapatite.

### Example 8

The following formulations were tested to determine the substantivity of cetylpyridinium chloride to HAP that had been treated with the test compounds in a separate step, prior to exposing the HAP to the cetylpyridinium chloride: water (control); phytic acid; sodium phosphate (NaH2PO4); and tin/phytic acid/phosphate (Sn-PaP). The phytic acid was used at 0.0015 M and prepared from a 0.045 M stock solution by removing a 5 ml aliquot to a vial and diluting with 10 ml of water. The pH of this solution was 2.5. The tin/phytic acid solution in 0.5 M phosphate buffer was prepared by weighing 0.0051 ± 0.0001 g SnCl₂ into a vial and brought into solution by the addition of 5 ml of water. A 5 ml aliquot of 0.0045 M phytic acid and a 5 ml aliquot of 1.5 M phosphate buffer were added to the tin solution to produce a formulation with 0.0015 M Sn; 0.0015 M phytic acid; and 0.5 M phosphate.

The HAP was prepared as described under general experimental and was treated with 2 ml of the test solutions containing phytic acid as indicated above. The HAP solids were then washed with 2-three ml portions of water and then exposed to 2 ml of 0.0015 M CPC solution. The HAP suspensions were then washed using the extended wash procedure of 22, 63.5, 84.5 and 105 ml described in Example 7.

The substantivity of the formulations as measured by the glycolysis pH test are given in Table IX.

**TABLE IX**

| pH as a Function of HAP Washings | | | | |
|---|---|---|---|---|
| Volume of Wash (ml) | Water | Phytic Acid | NaH₂PO₄ | Sn-PaP* |
| 22 | 5.39 | 7.44 | 7.28 | 7.21 |
| 63.5 | 5.24 | 7.81 | 5.19 | 7.39 |
| 84.5 | 5.28 | 7.52 | 5.26 | 7.51 |
| 105 | 5.32 | 7.47 | 5.25 | 7.41 |

| | | | | |
|---|---|---|---|---|
| *Sn-PaP = tin, phytic acid and phosphate | | | | |

The results show that the addition of tin does not interfere with the beneficial effect of phytic acid even with extended washing of the HAP. These results also show the beneficial effects of phytic acid and tin/phytic acid can be exerted by a two step exposure of the HAP.

### Example 9

In this trial, the preparation of HAP and the treatment of HAP with the test solutions were as previously described under general experimental. The following solutions were tested for HAP substantivity as measured by the glycolysis pH test:
A. Water (control); cetylpyridinium chloride (CPC);
B. Copper/phytic acid/sodium bicarbonate (CuPaB);
C. Cetylpyridinium chloride/sodium bicarbonate (CPCB);
D. N-tetradecyl-4-ethylpyridinium bromide (TDEP);
E. Copper/phytic acid/sodium bicarbonate/cetylpyridinium chloride (Cu-PaBC);
F. Phytic acid/sodium bicarbonate/cetylpyridinium chloride (PaBC);
G. Copper/phytic acid/sodium bicarbonate/N-tetradecyl-4-ethylpyridinium bromide (Cu-PaBT).

The concentration of the components being 0.0015 M except sodium bicarbonate at 0.5 M.

A 0.0045 M solution of N-tetradecyl-4-ethylpyridinium bromide was prepared by adding 0.0173 ± 0.0001 g of N-tetradecyl-4-ethylpyridinium bromide to a 10 ml volumetric flask and diluting to mark with water.

The results from this trial are given in Table X.

**TABLE X**

| pH as a Function of HAP Washings | | | | | | | | |
|---|---|---|---|---|---|---|---|---|
| | Samples | | | | | | | |
| VOLUME OF WASH (ml) | Water | CPC¹ | CuPaB² | CB³ | TDEP⁴ | Cu-PaBC⁵ | PaBC⁶ | Cu-PaBT⁷ |
| 3 | 5.23 | 7.71 | 8.01 | 8.78 | 7.82 | 8.62 | 8.78 | 8.73 |
| 13 | 5.27 | 7.80 | 6.05 | 8.53 | 5.42 | 8.36 | 8.50 | 8.39 |
| 21 | 5.30 | 6.73 | 5.82 | 5.73 | 5.32 | 8.23 | 8.37 | 8.26 |
| 31 | 5.34 | 5.43 | 5.84 | 6.05 | 5.39 | 8.19 | 8.25 | 8.14 |

| | | | | | | | | |
|---|---|---|---|---|---|---|---|---|
| ¹CPC = cetylpyridinium chloride; | | | | | | | | |
| ²CuPaB = copper, phytic acid and sodium bicarbonate; | | | | | | | | |
| ³CPCB = cetylpyridinium chloride and sodium bicarbonate; | | | | | | | | |
| ⁴TDEP = N-tetradecyl-4-ethylpyridinium bromide; | | | | | | | | |
| ⁵Cu-PaBC = copper, phytic acid, sodium bicarbonate and cetylpyridinium chloride; | | | | | | | | |
| ⁶PaBC = phytic acid, sodium bicarbonate, and cetylpyridinium chloride; | | | | | | | | |
| ⁷Cu-PaBT = copper, phytic acid, sodium bicarbonate and N-tetradecyl-4-ethylpyridinium bromide. | | | | | | | | |

The results show that sodium bicarbonate works as effectively in combination with phytic acid and cetylpyridinium chloride as does the phosphate buffer. In addition ethyltetradecylpyridinium bromide works as well as cetylpyridinium chloride as an antimicrobial in combination with phytic acid, copper and bicarbonate buffer.

### Example 10

An *in-vivo* study was conducted to determine the ability of the test formulations containing phytic acid to inhibit the experimental formation of gingivitis in Beagle dogs.

Purebred female Beagle dogs, 2-3 years old, with naturally occurring gingivitis were randomly divided into groups of four animals each. After 14 days of adaptation, the teeth of the dogs were scaled to remove supragingival calculus and polished. One week following the prophylaxes, during which time oral care was maintained by brushing, a baseline gingivitis index was obtained as measured by the procedure of Loe, *J. Periodontol*., 38, 610 (1967) and Loe and Silness, *Acta Odont Scand*, 21, 533 (1963). After the initial gingivitis index reading, the teeth of each group were sprayed twice daily, five days per week, with approximately 10 ml of one of the following mouth rinses:
(A) cetylpyridinium chloride (CPC);
(B) zinc/phytic acid/cetylpyridinium chloride/phosphate;
(C) copper/phytic acid/cetylpyridinium chloride/bicarbonate;
(D) tin/phytic acid/cetylpyridinium chloride/phosphate;
(E) phytic acid/cetylpyridinium chloride/phosphate.

All components were present at a concentration of 0.0015 M except the phosphate and bicarbonate at 0.5 M and the metals which were at 0.0014 M.

After four weeks of treatment, the gingival index was again measured. The results given in Table XI showing the increase in the gingival index, show that the phytic acid/cetylpyridinium chloride/phosphate composition was the most effective composition of those tested for inhibiting deterioration of the gingival health. The phytic acid containing test solutions gave a lower increase in the gingival index when compared to the cetylpyridinium chloride alone.

**TABLE XI**

| Formulation^{a} | Change in Gingival Index over 4 weeks |
|---|---|
| CPC | 0.528 |
| Zn-phytic/CPC/phos | 0.479 |
| Cu-phytic/CPC/bicarb | 0.430 |
| Sn-phytic/CPC/phos | 0.349 |
| phytic/CPC/phos | 0.199 |

| | |
|---|---|
| ^{a}CPC = cetylpyridinium chloride; phytic = phytic acid; phos = phosphate; bicarb = bicarbonate | |

### Example 11 Retention of CPC on Hydroxyapatite

To separate tubes containing (1) 4.5 mM cetylpyridinium chloride (CPC); (2) 4.5 mM phytic acid, 4.5 mM CPC, and 0.5 M phosphate, pH 7.47; and (3) 4.5 mM CPC in 0.5 M phosphate was added 100 mg of washed hydroxyapatite (added as 1 ml of 100 mg/ml suspension in water). The suspensions were mixed for 10 minutes, centrifuged, a 200 µl aliquot of the supernatant removed and diluted with 3.0 ml of water, and the absorbence of the diluted aliquot measured at 260 nanometers.

The supernatant remaining in each tube was carefully removed and the remaining hydroxyapatite resuspended in 3.0 ml of water and mixed for 10 minutes. The suspensions were again centrifuged and a 200 µl aliquot of the supernatant removed, diluted and ultraviolet absorbence measured at 260 nanometers. The supernatant was discarded after the absorbence reading. The procedure of resuspending the hydroxyapatite in 3 ml of water, mixing, centrifuging and measuring the absorbence of the supernatant was repeated an additional six times. By knowing how much CPC was adsorbed onto the hydroxyapatite and how much was being removed in each wash, the number of washes necessary to completely remove the CPC from the hydroxyapatite was estimated. The results from this trial are given in Table XII.

**TABLE XII**

| Sample | Percent of *CPC initially adsorbed onto hydroxyapatite | Number of water washes calculated to remove all CPC from hydroxyapatite |
|---|---|---|
| CPC | 37 | 14 |
| Phytic acid/CPC/Phosphate | 75 | 24 |
| Phosphate/CPC | 25 | 14 |

| | | |
|---|---|---|
| *CPC = cetylpyridinium chloride | | |

The results show that a compound of the present invention containing C-O-P bonds, such as phytic acid, increases the amount of CPC which is adsorbed onto the hydroxyapatite and causes the CPC to be desorbed at a lower rate.

### Example 12 Compatibility of Calcium with Phytic Acid and CPC in the Presence of Phosphate

To determine the compatibility of metal ions with phytic acid and cetylpyridinium chloride (CPC) in the presence of a phosphate buffer, a 0.0012 M solution of phytic acid was prepared in 0.5 M phosphate buffer (pH 7.32) containing 0.0015 M CPC. To separate 100 µl aliquots of this solution was added 15, 30, 45, 60 or 75 µl of a 0.01 M calcium chloride solution. This gave varying ratios of calcium to phytic acid in the presence of CPC in phosphate buffer. The results are given in Table XIII and show the compatibilizing effect of phosphate buffer on CPC/phytic acid solutions when exposed to calcium ions.

**TABLE XIII**

| µl of 0.01 M CaCl₂ | Molar ratio of Phytic acid:Ca:CPC* | Observation |
|---|---|---|
| 15 | 1:1.25:1.25 | clear |
| 30 | 1:2.5:1.25 | clear |
| 45 | 1:3.75:1.25 | clear |
| 60 | 1:5:1.25 | slightly turbid |
| 75 | 1:6.25:1.25 | turbid |

| | | |
|---|---|---|
| *CPC = cetylpyridinium chloride | | |

### Example 13 Effect of Surfactants on Maintaining CPC and Phytate Acid in Solution

Various amounts of the nonionic surfactants Polysorbate 80 (ICI Americas Inc., Wilmington, DE) and Poloxamer 407 (BASG Wyandotte Corp., Parsippany, NJ) and the anionic surfactant Hamposyl L-30 (WR Grace & Co., CT) were dissolved in 3.0 mM CPC solution. Polysorbate 80 is a polyethylene oxide sorbitan ester having a molecular formula of C₆₄H₁₂₅O₂₆, an approximate molecular weight of 1309 daltons, and is available as Tween™ 80. Poloxamer 407 is a poly(oxyethylene), poly(oxypropylene) block polymer commercially available as Pluronic™ F127 from BASF and has a molecular weight of approximately 12,600 daltons. Hamposyl L-30 is a surfactant available from W. R. Grace Company and contains by weight approximately 30 percent sodium lauryl sarcosinate, 68 percent water and 1 to 2 percent sodium laurate. Equal volumes of each of the surfactant/CPC solutions and 3.0 mM sodium phytate (pH=7) were then mixed in small vials. The weight percent of the surfactants present in the samples ranged from zero (control) to 0.4 percent. The vials were capped and shaken and visually monitored for about 30 minutes. The contents of each vial was then transferred to a separate plastic tube and centrifuged. The supernatant was then filtered through a 0.2 micrometer nylon syringe filter. A 200 µl portion of the filtrate was dissolved in 3.0 ml of water and the UV absorbence determined at 260 nm and compared against a similar aliquot of 1.5 mM CPC. The results for Polysorbate 80 indicated the lowest level of surfactant added to maintain solubility of the CPC and phytic acid under these conditions was about 0.1 weight percent; for Poloxamer 407 and Hamposyl L-30, the lowest level was about 0.2 weight percent.

### Example 14 Substantivity of Phytate/CPC Solutions Compatibilized by Surfactants

The preparation of the HAP and the treatment of the HAP with test solutions was as previously described in the general experimental except the amount of HAP used was reduced to 333 µl of 100 mg/ml suspension and the volume of water washes increased to give treated HAP samples washed with 22, 63.5, 84.5 and 105 ml of water. The following formulations from Example 13 were tested to determine the substantivity of CPC to HAP using this test:
A. 1.5 mM CPC
B. The filtered 0.1 weight percent Polysorbate 80.
C. The filtered 0.2 weight percent Polysorbate 80.
D. The filtered 0.3 weight percent Polysorbate 80.
E. The filtered 0.4 weight percent Polysorbate 80.
F. The filtered 0.5 weight percent Polysorbate 80.
G. The filtered 0.2 weight percent Hamposyl L-30.
H. The filtered 0.3 weight percent Hamposyl L-30.
I. The filtered 0.2 weight percent Poloxamer 407.
J. The filtered 0.3 weight percent Poloxamer 407.

The pH after incubation as a function of HAP washings for these solutions in this test are shown in Table XIV.

**TABLE XIV**

| pH as a Function of HAP Washings | | | | | | | | | | |
|---|---|---|---|---|---|---|---|---|---|---|
| Volume of Water Wash | A | B | C | D | E | F | G | H | I | J |
| 22 | 4.92 | 7.22 | 7.15 | 7.12 | 7.02 | 7.09 | 4.82 | 4.36 | 7.22 | 7.16 |
| 63.5 | 4.86 | 7.21 | 7.17 | 7.14 | 7.14 | 7.12 | 4.54 | 4.49 | 7.27 | 7.21 |
| 84.5 | 4.93 | 7.21 | 7.17 | 7.17 | 7.14 | 7.14 | 5.37 | 5.43 | 7.25 | 7.22 |
| 105 | 4.94 | 7.23 | 7.19 | 7.18 | 7.19 | 7.17 | 4.89 | 5.19 | 7.25 | 7.24 |

Additionally, 50 µl aliquots of the HAP samples were taken at the various stages of water washing, dissolved in 3.0 ml of 3 N HCl and analyzed by UV for CPC content by the degree of absorption at 260 nm. The results from this analysis are shown in Table XV.

**TABLE XV**

| UV Absorbance or Dissolved HAP from Washing Experiment Which Quantifies the Amount of CPC Still Present on the HAP | | | | |
|---|---|---|---|---|
| | | UV Absorbance | | (ave of 2) |
| | 22 ml wash | 63.5 ml wash | 84.5 ml wash | 105 ml wash |
| A | 0.0036 | 0.0031 | 0 | 0.0011 |
| B | 0.0666 | 0 | 0.0533 | 0.0380 |
| C | 0.0622 | 0.0462 | 0.0488 | 0.0378 |
| D | 0.0556 | 0.0533 | 0.0488 | 0.0362 |
| E | 0.0533 | 0.0467 | 0.0436 | 0.0338 |
| F | 0.0489 | 0.0444 | 0.04 | 0.0331 |
| G | 0.0084 | 0.0044 | 0.0018 | 0.0011 |
| H | 0.0049 | 0.0031 | 0.0089 | 0.0011 |
| I | 0.0778 | 0.0489 | 0.04 | 0.0216 |
| J | 0.0689 | 0.0556 | 0.048 | 0.0249 |

These results show that Polysorbate 80 (Samples B through F) and Poloxamer 407 (Samples I and J) can both maintain the CPC and phytic acid in solution and do not substantially interfere with the enhanced substantivity attributable to the presence of phytic acid. On the other hand, Hamposyl L-30 (Samples G and H) is capable of keeping the phytic acid and CPC mixture in solution, but interferes in the phytic acid mediated enhanced substantivity.

### Example 15 Use of Sodium Bicarbonate to Maintain Phytate and TDEP in Solution

Aliquots of a 1.5 M solution of sodium bicarbonate were placed in dram (3.7 ml) vials and diluted with water to 1 ml total volume. A 1.00 ml aliquot of 4.5 mM N-tetradecyl-4-ethylpyridinium bromide (TDEP) aqueous solution and a 1.00 ml aliquot of a 4.5 mM sodium phytate solution were added to each vial to give 1.5 mM sodium phytate, 1.5 mM TDEP and various levels of sodium bicarbonate (between zero and 2.5 weight percent). The caps were replaced, the vials shaken and then visually observed after standing at room temperature (16°C to 25°C) for 30 minutes. The observed turbidity is given in Table XVI and indicates that 1.64 weight percent of sodium bicarbonate is needed to maintain the sodium phytate and TDEP in solution.

**TABLE XVI**

| Observations of Interaction Between Sodium Phytate and TDEP in Various Levels of Sodium Bicarbonate | |
|---|---|
| Weight Percent NaHCO₃ | Visual Observation |
| 0 | Very Turbid |
| 0.82 | Turbid |
| 1.07 | Slightly Turbid |
| 1.31 | Less Turbid |
| 1.64 | Clear |
| 2.46 | Clear |

### Example 16 Use of Polysorbate to Maintain Phytate and TDEP in Solution

Aliquots of a 1.5 weight percent of Polysorbate 80 were placed in dram (3.7 ml) vials and diluted to 1.00 ml total volume with water. A 1.00 ml aliquot of 4.5 mM of N-tetradecyl-4-ethylpyridinium bromide (TDEP) aqueous solution and 1.00 ml aliquot of a 4.5 mM sodium phytate solution were added to the vials containing Polysorbate to give 1.5 mM sodium phytate, 1.5 mM TDEP in various levels of Polysorbate 80 (between zero and 0.5 weight percent). The caps were replaced, the vials shaken and the solutions visually monitored over time. The contents of each vial were then transferred to a plastic tube, centrifuged and the supernatant filtered through a 0.2 µm nylon syringe filter. A 200 µl portion of the filtrate was dissolved in 3.00 ml of water and the UV absorbence determined at 260 nm and compared to a similar sample of 1.5 mM TDEP. The results from the visual observations and the percent of original TDEP remaining in solution after filtering are given in Table XVII.

**TABLE XVII**

| Observations and UV Absorption Data From the Interaction of Sodium Phytate and TDEP in Polysorbate 80 | | |
|---|---|---|
| Weight Percent Polysorbate 80 | Visual Observation | Percent of Original TDEP in Solution after Filtering |
| 0 | Turbid | 0 |
| 0.05 | Slightly turbid | 86 |
| 0.075 | Clear | 100 |
| 0.10 | Clear | 96 |
| 0.20 | Clear | 102 |
| 0.30 | Clear | 99 |
| 0.40 | Clear | 98 |

The results indicate that about 0.075 weight percent of Polysorbate maintained the phytate and TDEP in solution as visually observed and as indicated by UV absorbence.

### Example 17 Use of Hamposyl to Maintain Phytate and TDEP in Solution

Aliquots of a 1.5 weight percent solution of Hamposyl L-30 were placed in dram (3.7 ml) vials and diluted with water to 1 ml total volume. A 1.00 ml aliquot of 4.5 mM of N-tetradecyl-4-ethylpyridinium bromide (TDEP) aqueous solution and a 1.00 ml aliquot of 4.5 mM sodium phytate solution were added to give 1.5 mM sodium phytate and 1.5 mM TDEP in various levels of Hamposyl L-30 (between zero and 0.5 weight percent). The caps were replaced, the vials shaken, and the solutions visually monitored over time (for approximately 1 hour). The contents of each vial were transferred to a separate plastic tube, centrifuged and the supernatant filtered through a 0.2 µm nylon syringe filter. A 200 µl portion of the filtrate was dissolved in 3.00 ml of water and the UV absorbence determined at 260 nm and compared to a similar sample of 1.5 mM TDEP. The results from the visual observations and percent of original TDEP remaining in solution are given in Table XVIII.

**TABLE XVIII**

| Observations and UV Absorption Data From the Interaction of Sodium Phytate and TDEP in Hamposyl L-30 | | |
|---|---|---|
| Weight Percent Hamposyl L-30 | Visual Observation | Percent of Original TDEP in Solution after Filtering |
| 0 | Turbid | 0 |
| 0.10 | Slightly turbid | 82 |
| 0.20 | Clear | 98 |
| 0.30 | Clear | 100 |
| 0.40 | Clear | 101 |
| 0.50 | Clear | 99 |

The results show that the amount of Hamposyl L-30 required to maintain the phytate and TDEP in solution is above 0.1 weight percent.

### Example 18 Use of Poloxamer to Maintain Phytate and TDEP in Solution

Aliquots of a 1.5 weight percent solution of Poloxamer 407 were placed in dram (3.7 ml) vials and diluted with water to 1 ml total volume. A 1.00 ml aliquot of 4.5 mM of N-tetradecyl-4-ethylpyridinium bromide (TDEP) aqueous solution and a 1.00 ml aliquot of 4.5 mM sodium phytate solution were added to give 1.5 mM sodium phytate and 1.5 mM TDEP in various levels of Poloxamer 407 (between zero and 0.5 weight percent). The caps were replaced, the vials shaken, and the solutions visually monitored over time (for approximately 1 hour). The contents of each vial were transferred to a separate plastic tube, centrifuged and the supernatant filtered through a 0.2 µm nylon syringe filter. A 200 µl portion of the filtrate was dissolved in 3.00 ml of water and the UV absorbence determined at 260 nm and compared to a similar sample of 1.5 mM TDEP. The results from the visual observations and percent of original TDEP remaining in solution are given in Table XIX.

**TABLE XIX**

| Observations and UV Absorption Data From the Interaction of Sodium Phytate and TDEP in Poloxamer 407 | | |
|---|---|---|
| Weight Percent Poloxamer 407 | Visual Observation | Percent of Original TDEP in Solution after Filtering |
| 0 | Turbid | 0 |
| 0.10 | Slightly turbid | 29 |
| 0.20 | Slightly turbid | 64 |
| 0.30 | Less turbid | 92 |
| 0.40 | Clear | 97 |
| 0.50 | Clear | 98 |

The results indicate that the amount of Poloxamer L-30 required to maintain the phytate and TDEP in solution is above 0.10 weight percent.

### Example 19 Use of Polysorbate to Maintain Phytate and Sanguninarine in Solution

Aliquots of a 10 weight percent solution of Polysorbate 80 were placed in dram (3.7 ml) vials and diluted with water to 1 ml total volume. A 1.00 ml aliquot of 4.5 mM of Sanguinarine aqueous solution and 1.00 ml of 4.5 mM of sodium phytate were added to each vial to give 1.5 mM sodium phytate and 1.5 mM Sanguinarine in various levels of Polysorbate 80 (between zero and 3.3 weight percent). The caps were replaced, the vials shaken and then visually-monitored for one hour. The recorded visual observations are given in Table XX and indicate that greater than 1.67 weight percent of Polysorbate 80 is necessary to maintain the phytate and sanguinarine in solution.

**TABLE XX**

| Observations and UV Absorption Data from the Interaction of Sodium Phytate and Sanguinarine in Polysorbate 80 | |
|---|---|
| Weight Percent Polysorbate 80 | Visual Observation |
| 0 | Turbid |
| 0.40 | Turbid |
| 1.67 | Less turbid |
| 3.33 | Clear |

### Example 20 Use of Hamposyl to Maintain Phytate and Sanguinarine in Solution

Aliquots of a 3 weight percent solution of Hamposyl L-30 were placed in dram (3.7 ml) vials and diluted with water to 1 ml total volume. A 1.00 ml aliquot of 4.5 mM of Sanguinarine aqueous solution and 1.00 ml of 4.5 mM of sodium phytate were added to each vial to give 1.5 mM sodium phytate and 1.5 mM Sanguinarine in various levels of Hamposyl L-30 (between zero and 0.8 weight percent). The caps were replaced, the vials shaken and then visually-monitored for one hour. The recorded visual observations are given in Table XXI and indicate that greater than 0.67 weight percent of Hamposyl L-30 is necessary to maintain the sanguinarine and phytate in solution.

**TABLE XXI**

| Observations and UV Absorption Data from the Interaction of Sodium Phytate and Sanguinarine in Hamposyl L-30 | |
|---|---|
| Weight Percent Hamposyl L-30 | Visual Observation |
| 0 | Turbid |
| 0.40 | Turbid |
| 0.67 | Less turbid |
| 0.75 | Clear |

### Example 21 Substantivity of TDEP/Phytic Acid Solutions Compatibilized by Bicarbonate or Surfactants

The preparation of the HAP and the treatment of the HAP with test solutions was as previously described in Example 14. The following formulations were tested to determine the substantivity of TDEP to HAP using this test:
A. 1.5 mM cetylpyridinium chloride (CPC)
B. 1.5 mM N-tetradecyl-4-ethylpyridinium bromide (TDEP)
C. 1.5 mM TDEP in 0.5 M sodium bicarbonate (TDEP B)
D. A 1.5 mM TDEP in 0.5 M sodium bicarbonate containing 1.5 mM phytic Acid (TDEPPaB)
E. 1.5 mM TDEP containing 0.4 percent Polysorbate 80 (TDEPPoly)
F. 1.5 mM TDEP containing 0.4 percent Poloxamer 407 (TDEPPolx)
G. Solution E but also containing 1.5 mM phytic acid (TDEPPaPoly)
H. 1.5 mM TDEP containing 0.4 percent Hamposyl L-30 and 1.5 mM phytic acid (TDEPPaHamp)
I. Solution F but also containing 1.5 mM phytic acid (TDEPPaPolx)
The pH as a function of HAP washings for these solutions in this test are shown in Table XXII.

**TABLE XXII**

| pH as a Function of HAP Washings | | | | | | | | | |
|---|---|---|---|---|---|---|---|---|---|
| Volume of wash (ml) | CPC¹ | TDEP² | TDEPB³ | TDEP-PaB⁴ | TDEP-Poly⁵ | TDEP-Polx⁶ | TDEPPa-Poly⁷ | TDEPPa-Hamp⁸ | TDEPPa-Polx⁹ |
| 22 | 7.09 | 4.79 | 4.98 | 5.09 | 4.97 | 4.97 | 7.24 | 7.18 | 7.11 |
| 63.5 | 4.67 | 4.84 | 4.79 | 4.79 | 4.99 | 4.99 | 7.25 | 4.81 | 7.12 |
| 84.5 | 4.78 | 4.85 | 4.89 | 4.78 | 4.98 | 5.01 | 7.25 | 4.82 | 7.12 |
| 105 | 4.81 | 4.87 | 4.85 | 4.76 | 4.98 | 5.01 | 7.26 | 4.84 | 4.78 |

| | | | | | | | | | |
|---|---|---|---|---|---|---|---|---|---|
| ¹CPC = cetylpyridinium chloride | | | | | | | | | |
| ²TDEP = N-tetradecyl-4-ethylpyridinium bromide | | | | | | | | | |
| ³TDEPB = TDEP and sodium bicarbonate | | | | | | | | | |
| ⁴TDEP-PaB = TDEP, phytic acid and sodium bicarbonate | | | | | | | | | |
| ⁵TDEP-Poly = TDEP and polysorbate 80 | | | | | | | | | |
| ⁶TDEP-Polx = TDEP and poloxamer 407 | | | | | | | | | |
| ⁷TDEPPa-Poly = TDEP, phytic acid and polysorbate 80 | | | | | | | | | |
| ⁸TDEPPa-Hamp = TDEP, phytic acid and hamposyl L30 | | | | | | | | | |
| ⁹TDEPPa-Polx = TDEP, phytic acid and poloxamer 407 | | | | | | | | | |

Additionally, 50 µl aliquots of the HAP samples were taken at the various water washing steps, dissolved in 3.0 ml of 3 N HCl and analyzed by UV for CPC content by the degree of absorption at 260 nm. The results from this analysis are given in Table XXIII.

**TABLE XXIII**

| Volume of Wash (ml) | CPC | TDEP² | TDEPB³ | TDEP PaB⁴ | TDEP-Poly⁵ | TDEP-Polx⁶ | TDEPPA Poly⁷ | TDEPPA-Hamp⁸ | TDEPA-Polx⁹ |
|---|---|---|---|---|---|---|---|---|---|
| 22 | .0051 | .0025 | .0024 | .0038 | .0000 | .0000 | .0544 | .0127 | .0771 |
| 63.4 | .0046 | .0000 | .0024 | .0016 | .0000 | .0000 | .0344 | .0000 | .0179 |
| 84.5 | .0000 | .0000 | .0024 | .0000 | .0000 | .0000 | .0123 | .0000 | .0037 |
| 10.5 | .0000 | .0000 | .0000 | .0000 | .0000 | .0000 | .0060 | .0000 | .0006 |

| | | | | | | | | | |
|---|---|---|---|---|---|---|---|---|---|
| ¹CPC = cetylpyridinium chloride | | | | | | | | | |
| ²TDEP = N-tetradecyl-4-ethylpyridinium bromide | | | | | | | | | |
| ³TDEPB = TDEP and sodium bicarbonate | | | | | | | | | |
| ⁴TDEP-PaB = TDEP, phytic acid and sodium bicarbonate | | | | | | | | | |
| ⁵TDEP-Poly = TDEP and Polysorbate 80 | | | | | | | | | |
| ⁶TDEP-Polx = TDEP and Poloxamer 407 | | | | | | | | | |
| ⁷TDEPPa-Poly = TDEP, phytic acid and Polysorbate 80 | | | | | | | | | |
| ⁸TDEPPa-Hamp = TDEP, phytic acid and Hamposyl L30 | | | | | | | | | |
| ⁹TDEPPa-Polx = TDEP, phytic acid and Poloxamer 407 | | | | | | | | | |

These results in conjunction with the results on the solubility of phytic acid and TDEP in the presence of bicarbonate or surfactants indicates that at certain concentrations, bicarbonate, Polysorbate 80, Hamposyl L-30, and Poloxamer 407 can all maintain TDEP and phytic acid in solution but only Poloxamer 407 and Polysorbate 80 allow the phytic acid to enhance the substantivity of TDEP to hydroxyapatite.

### Example 22 Substantivity of Phytic Acid Combinations with Sanguinarine Compatibilized by Surfactants

The preparation of the HAP and the treatment of the HAP with test solutions was as previously described in Example 14. the following formulations were tested to determine the substantivity of Sanguinarine to HAP:
A. 1.5 mM cetylpyridinium chloride (CPC)
B. 1.5 mM Sanguinarine (Sa)
C. 1.5 mM Sanguinarine containing 3.33 percent Polysorbate 80 (SaPoly)
D. Solution C but also containing 1.5 mM phytic acid (SaPa Poly)
E. 1.5 mM Sanguinarine containing 0.75 percent Hamposyl L-30 (SaHamp), (The mixture was filtered immediately before use due to a precipitate being formed.)
F. Solution E but also containing 1.5 mM phytic acid with all components being soluble (SaPaHamp).
The pH as a function of HAP washings for these solutions is given in Table XXIV.

**TABLE XXIV**

| pH as a Function of HAP Washings | | | | | | |
|---|---|---|---|---|---|---|
| Volume of Wash (ml) | CPC¹ | Sa² | SaPoly³ | SaPaPoly⁴ | SaHamp⁵ | SaPaHamp⁶ |
| 22 | 7.18 | 5.26 | 5.04 | 6.92 | 7.23 | 7.31 |
| 63.5 | 4 .89 | 5.08 | 5.07 | 4.81 | 5.23 | 4.98 |
| 84.5 | 4.96 | 5.01 | 5.12 | 4.84 | 5.25 | 4.97 |
| 105 | 4.98 | 5.11 | 5.09 | 4.89 | 5.31 | 4.98 |

| | | | | | | |
|---|---|---|---|---|---|---|
| ¹CPC = cetylpyridinium chloride | | | | | | |
| ²Sa = Sanguinarine | | | | | | |
| ³SaPoly = Sanguinarine and Polysorbate 80 | | | | | | |
| ⁴SaPaPoly = Sanguinarine, phytic acid and Polysorbate 80 | | | | | | |
| ⁵SaHamp = Sanguinarine and Hamposyl L-30 | | | | | | |
| ⁶SaPaHamp = Sanguinarine, phytic acid and Hamposyl L-30 | | | | | | |

Additionally, 50 µl aliquots of the HAP samples were taken at the various stages of water washing, dissolved in 3.0 ml of 3 N HCl and analyzed by UV for sanguinarine content by the degree of absorption at 260 nm. The results from this analysis are given in Table XXV.

**TABLE XXV**

| Volume of Wash (ml) | CRC¹ | Sa² | SaPoly³ | SaPaPoly⁴ | SaHamp⁵ | SaPaHamp⁶ |
|---|---|---|---|---|---|---|
| 22 | .0011 | .0007 | .0013 | .0186 | .0365 | .1489 |
| 63.5 | .0000 | .0019 | .0000 | .0000 | .0022 | .0034 |
| 84.5 | .0000 | .0013 | .0000 | .0000 | .0016 | .0022 |
| 105 | .0000 | .0024 | .0000 | .0000 | .0000 | .0013 |

| | | | | | | |
|---|---|---|---|---|---|---|
| ¹CPC = cetylpyridinium chloride | | | | | | |
| ²Sa = Sanguinarine | | | | | | |
| ³SaPoly = Sanguinarine and Polysorbate 80 | | | | | | |
| ⁴SaPaPoly = Sanguinarine; phytic acid and Polysorbate 80 | | | | | | |
| ⁵SaHamp = Sanguinarine and Hamposyl L-30 | | | | | | |
| ⁶SaPaHamp = Sanguinarine; phytic acid and Hamposyl L-30 | | | | | | |

These results, in conjunction with the results on the solubility of phytic acid and sanguinarine in the presence of Polysorbate 80 and Hamposyl L-30, indicate that at certain concentrations Polysorbate 80 and Hamposyl L-30 surfactants can both compatibilize sanguinarine and phytic acid. Additionally, both Polysorbate 80 and Hamposyl L-30 allows the phytic acid to enhance the substantivity of sanguinarine to hydroxyapatite.

### Example 23

A formulation containing cetylpyridinium chloride, sodium phytic and sodium bicarbonate for human trials is given in Table XXVI. This formulation was tested with water and a mouthwash which contained CPC but did not contain phytic acid. Twenty four subjects, 19 to 57 years of age were enroled in a crossover design with 8 week duplicate measurements. At each weekly session subjects received two baseline measurements (pretreatment), then rinsed with mouthwash, and two post-treatment measurements approximately 8 hours later. Supra-gingival plaque was completely removed from preselected Ramford teeth for microbial analysis pretreatment and 8 hours post treatment. Analyses were performed on these plaque samples for aerobes, anaerobes, and fusobacteria. The ratio of the log post-treatment bacterial counts to pretreatment bacterial counts (baseline), times one hundred, is given in Table XXVII.

**TABLE XXVI**

| Component | Grams | Approximate Weight Percent |
|---|---|---|
| Cetyl pyridinium Chloride | 3.288 | 0.055 |
| Sodium Bicarbonate | 252 | 4.2 |
| Citric Acid | 30 | 0.5 |
| Glycerin | 300 | 5 |
| Mint Fresh Flavor | 30 | 0.5 |
| Menthol Natural | 1.2 | 0.02 |
| Saccharin Powder | 1.2 | 0.02 |
| Propylene Glycol | 150 | 2.5 |
| Sodium Phytate (43 weight % solution) | 13.6 | 0.23 |
| Purified Water | To Make 6 Liters Volume | 87 |

**TABLE XXVII**

| Bacteria Type | Treatment with Mouthwash of Example 25 | Treatment with Commercially Available CPC Containing Mouthwash | Treatment with Water |
|---|---|---|---|
| Aerobes | 10.0 | 15.9 | 27.6 |
| Anaerobes | 12.5 | 21.9 | 27.7 |
| Fiusobacteria | 19.6 | 29.0 | 21.0 |

Multiple range analyses showed that the results using the mouthwash containing CPC and phytic acid was the only one significantly different from water. These results indicate that the addition of phytic acid to CPC in the presence of a compatibilizing agent (bicarbonate buffer in this formulation) can exert prolonged antibacterial effects in supragingival plaque.

### Example 24 Analyses for CPC content in Plaque samples gathered in the Trial described in Example 23.

During the trial described in Example 23, plaque was harvested after 8 hours overnight from the pre-selected Ramford teeth. Additionally, 2 hours after treatment plaque was harvested from the opposite Ramford teeth. Lastly, 4 hours post-treatment samples of plaque were collected from the four second molars. All the plaque samples were placed in 1 ml of Ringers solution and later analyzed for CPC content using HPLC. For the three time points (2, 4, and 8 hour) post-treatment, the average CPC recovery for subjects using the Formulation of Example 23 was about twice that as compared to the CPC recovery for subjects using a mouthrinse containing CPC but containing no phytic acid.

### Example 25 Sulfur Analysis of Breath of Human Subjects Using the Formulation of Example 23

Volatile sulfur compounds (VSC) were measured using a portable industrial sulphide monitor (model 1170, 0.5 ppm full-scale, available from Interscan Corp., Chatsworth, CA) as described by Rosenberg et al., *J. Dental Research*, 70, 1436-1440 (1991). The four day trial consisted of six volunteers that crossed over randomly from a mouthwash containing CPC (but no phytic acid) to the formulation of Example 23 (containing CPC/phytic acid/bicarbonate). The volunteers brushed their teeth at night using their usual toothpaste and toothbrush followed by rinsing for 60 seconds with 10 ml of mouthwash. The volunteers then retired for the night. The next morning the volunteers refrained from eating and drinking and minimized talking before getting their breath measured on the portable sulphide monitor (PSM). The breath measurement was taken on the PSM after the volunteers kept their mouths closed for three minutes. Five consecutive peak values on the PSM were recorded for each volunteer for each morning reading. The morning readings were taken on consecutive days of the week (Tuesday through Friday). The average morning breath readings after use of the formula of Example 23 showed about a 50 percent reduction in volatile sulfur components as measured on the PSM when compared to average morning breathe readings after use of a mouthwash containing CPC at similar levels but containing no phytic acid.

### Comparative A Use of Sodium Bicarbonate to Maintain Phytate and Sanguinarine in Solution

The procedure of Example 15 was repeated substituting a 4.5 mM sanguinarine for the TDEP solution and the amount of bicarbonate solution being between zero and 8 percent by weight. The results from the trial are given in Table XXVIII and show that even at 8 weight percent sodium bicarbonate, the phytic acid and sanguinarine were not completely soluble.

**TABLE XXVIII**

| Observations of Interaction Between Sodium Phytate and Sanguinarine in Various Levels of Sodium Bicarbonate | |
|---|---|
| Weight Percent NaHCO₃ | Visual Observation |
| 0 | Very Turbid |
| 0.82 | Turbid |
| 1.64 | Turbid |
| 2.46 | Turbid |
| 3.28 | Turbid |
| 4.10 | Turbid |
| 8.02 | Turbid |

### Comparative B Use of Poloxamer to Maintain Phytate and Sanguinarine in Solution

Aliquots of a 10 weight percent solution of Poloxamer 407 were placed in dram (3.7 ml) vials and diluted with water to 1 ml total volume. A 1.00 ml aliquot of 4.5 mM of Sanguinarine aqueous solution and 1.00 ml of 4.5 mM of sodium phytate were added to each vial to give 1.5 mM sodium phytate and 1.5 mM Sanguinarine in various levels of Poloxamer 407 (between zero and 3.3 weight percent). The caps were replaced, the vials shaken and then visually-monitored for one hour. The recorded visual observations are given in Table XXIX and indicate that even at 3.33 weight percent Poloxamer, the sanguinarine and phytate did not remain in solution.

**TABLE XXIX**

| Observations and UV Absorption Data from the Interaction of Sodium Phytate and Sanguinarine in Poloxamer 407 | |
|---|---|
| Weight Percent Poloxamer 407 | Visual Observations |
| 0 | Turbid |
| 0.40 | Turbid |
| 1.67 | Turbid |
| 3.33 | Turbid |

Other embodiments of the invention will be apparent to those skilled in the art from a consideration of this specification or practice of the invention disclosed herein. It is intended that the specification and examples be considered as exemplary only, with the scope of the invention being indicated by the following claims.

## Claims

1. An oral composition comprising:
(a) from 0.001 to 10 percent by weight of one or more compounds having C-O-P bonds selected from myo-inositol hexakis(dihydrogen phosphate), myo-inositol pentakis(dihydrogen phosphate), myo-inositol tetrakis(dihydrogen phosphate) or physiologically acceptable salts thereof;
(b) from 0.001 to 10 percent by weight of one or more cationic antimicrobial compounds;
(c) from 0.1 to 20 percent by weight of one or more compatibilizing agents; and
(d) an orally acceptable vehicle.

2. A composition of Claim 1 wherein the cationic antimicrobial compound is one or more quaternary ammonium compounds of Formula I 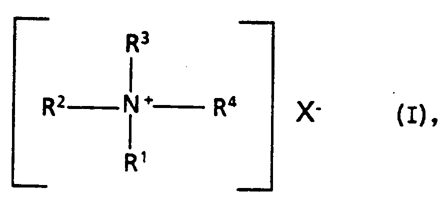 formula II 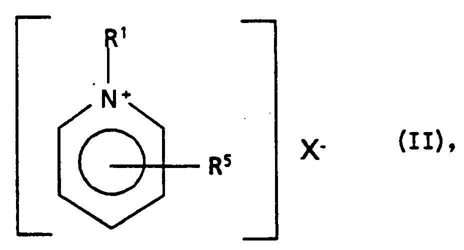 or a mixture thereof;
wherein
R¹ is a C₈-C₂₀ alkyl,
R² is benzyl or C₁-C₁₂ alkyl,
R³ and R⁴ are independently a C₁-C₇ alkyl or -(CH₂-CHOH-CH₂-O)ₙH wherein n is an integer from 1 to 6 inclusive,
R⁵ is -H, a C₁-C₇ alkyl or -(CH₂-CHOH-CH₂-O)ₙH wherein n is an integer from 1 to 6 inclusive, and
X⁻ is chloride, bromide, iodide or fluoride ion.

3. A composition of Claim 2 wherein the quaternary ammonium compound is cetylpyridinium chloride or N-tetradecyl-4-ethylpyridinium chloride.

4. A composition of Claim 1 wherein the cationic antimicrobial compound is sanguinarine.

5. A composition of Claim 1 wherein the compatibilizing agent is an anionic buffer selected from phosphate, acetate, borate, citrate, bicarbonate, gluconate, tartrate, sulfate and mixtures thereof.

6. A composition of Claim 5 wherein the C-O-P compound is myo-inositol hexakis(dihydrogen phosphate) or a physiologically acceptable salt thereof; the cationic antimicrobial compound is cetylpyridinium chloride; the compatibilizing agent is bicarbonate; and the orally acceptable vehicle is 70 to 99.9 percent by weight water or an alcohol-water mixture.

7. A composition of Claims 1 wherein the compatibilizing agent is a surfactant.

8. A composition of Claim 7 wherein the surfactant is a poly(oxyethylene), poly(oxypropylene) block polymer, a polyethylene oxide sorbitan ester, or a N-lauroyl sarcosine.

9. A composition according to any one of Claims 1 to 8 wherein the composition further contains a metal ion selected from copper, magnesium, tin, zinc, strontium, calcium and mixtures thereof, wherein the molar ratio of the metal ion to the compound having C-O-P bonds is from 4:1 to 1:4.

10. An oral composition comprising;
(a) from 0.001 to 10 percent by weight of one or more compounds having C-O-P bonds selected from is myo-inositol hexakis(dihydrogen phosphate), myo-inositol pentakis(dihydrogen phosphate), myo-inositol tetrakis(dihydrogen phosphate) and physiologically acceptable salts thereof;
(b) from 0.001 to 10 percent by weight of N-tetradecyl-4-ethylpyridinium chloride;
(c) from 0.05 to 20 percent by weight of a surfactant, wherein the surfactant is a polyethylene oxide sorbitan ester; and
(d) the remaining percent by weight is an orally acceptable vehicle.

11. A process for preparing an oral composition as defined in Claim 1 comprising the steps of
(a) dissolving the compound having C-O-P bonds and the compatibilizing agent in water; and
(b) dissolving the cationic antimicrobial compound or a solution of the cationic antimicrobial compound in the solution obtained from step (a),
wherein the pH of the solution is adjusted to between 6 and 8 after step (a) or step (b).

12. A kit for inhibiting the formation of dental calculus or dental plaque in a mammalian oral cavity comprising one or more compounds having C-O-P bonds in an orally acceptable vehicle, wherein the compound having C-O-P bonds is selected from myo-inositol hexakis(dihydrogen phosphate), myo-inositol pentakis(dihydrogen phosphate), myo-inositol tetrakis(dihydrogen phosphate) and physiologically acceptable salts thereof; a compatibilizing agent in an orally acceptable vehicle; and one or more cationic antimicrobial compounds in an orally acceptable vehicle; and a means to maintain the compound having C-O-P bonds separately from the cationic antimicrobial compound.

13. The kit of Claim 12 wherein the concentration of the compound containing C-O-P bonds is from 0.001 to 10 percent by weight of the orally acceptable vehicle, the concentration of the compatibilizing agent is from 0.001 to 20 percent by weight of the orally acceptable vehicle and the cationic antimicrobial compound is from 0.01 to 10 percent by weight of the orally acceptable vehicle.

14. A composition according to any one of Claims 1 to 10 for use in the treatment of dental calculus or dental plaque.

15. The use of a composition according to any one of Claims 1 to 10 for the manufacture of an oral composition for the treatment of dental calculus or dental plaque.

16. The use of an anionic buffer to stabilize a mixture of a compound having C-O-P bonds as defined in Claim 1 and one or more cationic antimicrobial compounds against precipitation from aqueous solution.

17. The use of a surfactant to stabilize a mixture of a compound having C-O-P bonds as defined in Claim 1 and one or more cationic antimicrobial compounds against precipitation from aqueous solution.

## Patentansprüche

1. Oralzusammensetzung, umfassend:
(a) von 0,001 bis 10 Gew.-% einer oder mehrerer Verbindungen mit C-O-P-Bindungen, ausgewählt aus Myo-Inositolhexakis(dihydrogenphosphat), Myo-Inositolpentakis(dihydrogenphosphat), Myo-Inositoltetrakis(dihydrogenphosphat) oder physiologisch verträglichen Salzen davon,
(b) von 0,001 bis 10 Gew.-% einer oder mehrerer kationischer antimikrobleller Verbindungen,
(c) von 0,1 bis 20 Gew.-% eines oder mehrerer Kompatibilisierungsmittel und
(d) ein oral verträgliches Vehikel.

2. Zusammensetzung nach Anspruch 1, worin die kationische antimikrobielle Verbindung eine oder mehrere quaternäre Ammoniumverbindungen der Formel I 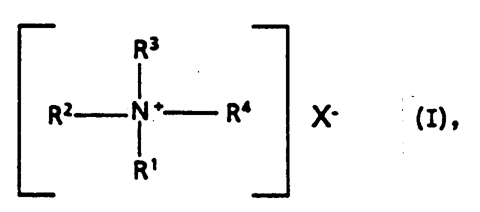 der Formel II 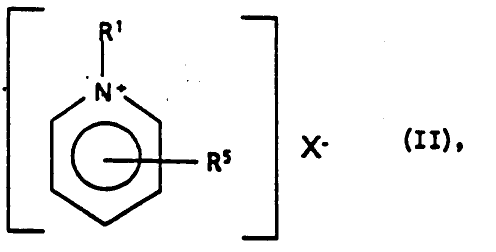 oder ein Gemisch davon ist,
worin
R¹ ein C₈-C₂₀-Alkyl ist,
R² Benzyl oder C₁-C₁₂-Alkyl ist,
R³ und R⁴ unabhängig ein C₁-C₇-Alkyl oder -(CH₂-CHOH-CH₂-O)ₙH sind, worin n eine ganze Zahl von 1 bis einschließlich 6 ist,
R⁵ -H, ein C₁-C₇-Alkyl oder -(CH₂-CHOH-CH₂-O)ₙH ist, worin n eine ganze Zahl von 1 bis einschließlich 6 ist, und
X⁻ ein Chlorid-, Bromid-, Jodid- oder Fluoridion ist.

3. Zusammensetzung nach Anspruch 2, worin die quaternäre Ammoniumverbindung Cetylpyridiniumchlorid oder N-Tetradecyl-4-ethylpyridiniumchlorid ist.

4. Zusammensetzung nach Anspruch 1, worin die kationische antimikrobielle Verbindung Sanguinarin ist.

5. Zusammensetzung nach Anspruch 1, worin das Kompatibilisierungsmittel ein anionischer Puffer ist, ausgewählt aus Phosphat, Acetat, Borat, Citrat, Bicarbonat, Gluconat, Tartrat, Sulfat und Gemischen davon.

6. Zusammensetzung nach Anspruch 5, worin die C-O-P-Verbindung Myo-Inositolhexakis(dihydrogenphosphat) oder ein physiologisch verträgliches Salz davon ist, die kationische antimikrobielle Verbindung Cetylpyridiniumchlorid ist, das Kompatibilisierungsmittel Bicarbonat ist und das oral verträgliche Vehikel 70 bis 99,9 Gewichtsprozent Wasser oder ein Alkohol-Wasser-Gemisch ist.

7. Zusammensetzung nach Anspruch 1, worin das Kompatibilisierungsmittel ein oberflächenaktives Mittel ist.

8. Zusammensetzung nach Anspruch 7, worin das oberflächenaktive Mittel ein Poly(oxyethylen)-Poly(oxypropylen)-Blockpolymer, ein Polyethylenoxidsorbitanester oder ein N-Lauroylsarcosin ist.

9. Zusammensetzung nach einem der Ansprüche 1 bis 8, worin die Zusammensetzung weiterhin ein Metallion enthält, ausgewählt aus Kupfer, Magnesium, Zinn, Zink, Strontium, Calcium und Gemischen davon, worin das molare Verhältnis des Metallions zu der Verbindung mit C-O-P-Bindungen von 4:1 bis 1:4 ist.

10. Oralzusammensetzung, umfassend
(a) von 0,001 bis 10 Gew.-% einer oder mehrerer Verbindungen mit C-O-P-Bindungen, ausgewählt aus Myo-Inositolhexakis(dihydrogenphosphat), Myo-Inositolpentakis(dihydrogenphosphat), Myo-Inositoltetrakis(dihydrogenphosphat) und physiologisch verträglichen Salzen davon,
(b) von 0,001 bis 10 Gew.-% N-Tetradecyl-4-ethylpyridiniumchlorid,
(c) von 0,05 bis 20 Gew.-% eines oberflächenaktiven Mittels, worin das oberflächenaktive Mittel ein Polyethylenoxidsorbitanester ist; und
(d) die verbleibenden Gewichtsprozente ein oral verträgliches Vehikel sind.

11. Verfahren zum Herstellen einer Oralzusammensetzung nach Anspruch 1, umfassend die Schritte des
(a) Lösens der Verbindung mit C-O-P-Bindungen und des Kompatibilisierungsmittels in Wasser und
(b) Lösen der kationischen antimikrobiellen Verbindung oder einer Lösung der kationischen antimikrobiellen Verbindung in der aus Schritt (a) erhaltenen Lösung,
worin der pH der Lösung auf zwischen 6 und 8 nach Schritt (a) oder Schritt (b) eingestellt wird.

12. Kit zum Inhibieren der Bildung von Zahnstein oder dentalem Plaque in einer Säugermundhöhle, umfassend eine oder mehrere Verbindungen mit C-O-P-Bindungen in einem oral verträglichen Vehikel, worin die Verbindung mit C-O-P-Bindungen ausgewählt ist aus Myo-Inositolhexakis(dihydrogenphosphat), Myo-Inositolpentakis(dihydrogenphosphat), Myo-Inositoltetrakis(dihydrogenphosphat) und physiologisch verträglichen Salzen davon, ein Kompatibilisierungsmittel in einem oral verträglichen Vehikel und eine oder mehrere kationische Verbindungen in einem oral verträglichen Vehikel und ein Mittel, um die Verbindung mit C-O-P-Bindung getrennt von der kationischen antimikrobiellen Verbindung zu halten.

13. Kit nach Anspruch 12, worin die Konzentration der Verbindung mit C-O-P-Bindungen von 0,001 bis 10 Gew.-% des oral verträglichen Vehikels ist, die Konzentration des Kompatibilisierungsmittels von 0,001 bis 20 Gew.-% des oral verträglichen Vehikels ist und die kationische antimikrobielle Verbindung von 0,01 bis 10 Gew.-% des oral verträglichen Vehikels ist.

14. Zusammensetzung nach einem der Ansprüche 1 bis 10 zur Verwendung bei der Behandlung von Zahnstein oder dentalem Plaque.

15. Verwendung einer Zusammensetzung nach einem der Ansprüche 1 bis 10 zur Herstellung einer Oralzusammensetzung für die Behandlung von Zahnstein oder dentalem Plaque.

16. Verwendung eines anionischen Puffers zum Stabilisieren eines Gemisches aus einer Verbindung mit C-O-P-Bindungen nach Anspruch 1 und einer oder mehrerer kationischer antimikrobieller Verbindungen gegen Präzipitation aus wäßriger Lösung.

17. Verwendung eines oberflächenaktiven Mittels zum Stabilisieren eines Gemischs aus einer Verbindung mit C-O-P-Bindungen nach Anspruch 1 und einer oder mehrerer kationischen antimikrobiellen Verbindungen gegen Präzipitation aus wäßriger Lösung.

## Revendications

1. Composition orale comprenant:
(a) de 0,001 à 10 pour-cent en poids d'un ou plusieurs composés ayant des liaisons C-O-P choisis parmi le hexakis(dihydrogénophosphate) de myo-inositol, le pentakis(dihydrogénophosphate) de myo-inositol, le tétrakis(dihydrogénophosphate) de myo-inosital et leurs sels acceptables sur le plan physiologique;
(b) de 0,001 à 10 pour-cent en poids d'un ou plusieurs composés cationiques antimicrobiens;
(c) de 0,1 à 20% en poids d'un ou plusieurs agents assurant la compatibilité; et
(d) un véhicule oralement acceptable.

2. Composition selon la revendication 1, dans laquelle le composé cationique antimicrobien est un ou plusieurs composés d'ammonium quaternaire de formule I 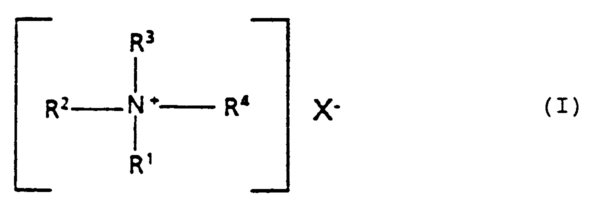 de formule II 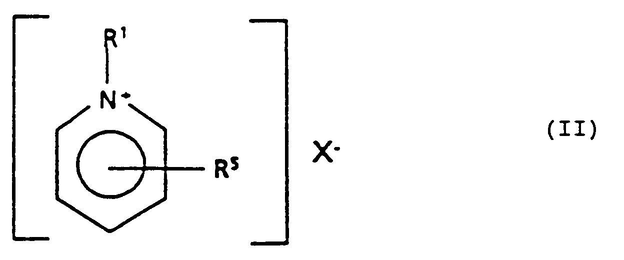 ou leur mélange;
formules dans lesquelles
R¹ est un radical alkyle en C₈-C₂₀,
R² est un radical benzyle ou un radical alkyle en C₁-C₁₂,
R³ et R⁴ sont indépendamment un radical alkyle en C₁-C₇ ou -(CH₂-CHOH-CH₂-O)ₙH où n est un nombre entier compris entre 1 et 6 inclus,
R⁵ représente -H, un radical alkyle en C₁-C₇ ou -(CH₂-CHOH-CH₂-O)ₙH où n est un nombre entier compris entre 1 et 6 inclus, et
X⁻ est un ion chlorure, bromure, iodure ou fluorure.

3. Composition selon la revendication 2, dans laquelle le composé ammonium quaternaire est le chlorure de cétyl-pyridinium ou le chlorure de N-tétradécyl-4-éthylpyridinium.

4. Composition selon la revendication 1, dans laquelle le composé cationique antimicrobien est la sanguinarine.

5. Composition selon la revendication 1, dans laquelle l'agent assurant la compatibilité est un tampon anionique choisi parmi les phosphate, acétate, borate, citrate, bicarbonate, gluconate, tartrate, sulfate et leurs mélanges.

6. Composition selon la revendication 5, dans laquelle le composé C-O-P est le hexakis(dihydrogénophosphate) de myo-inositol ou un sel de celui-ci acceptable sur le plan physiologique ; le composé cationique antimicrobien est le chlorure de cétylpyridinium; l'agent assurant la compatibilité est le bicarbonate; et le véhicule oralement acceptable comprend de l'eau ou un mélange alcool-eau à raison de 70 à 99,9 pour-cent en poids.

7. Composition selon la revendication 1, dans laquelle l'agent assurant la compatibilité est un tensioactif.

8. Composition selon la revendication 7, dans laquelle le tensioactif est un polymère séquencé de poly(oxyde d'éthylène), poly(oxyde de propylène), un ester sorbitan et de poly(oxyde d'éthylène), ou une N-lauroyl-sarcosine.

9. Composition selon l'une des revendications 1 à 8, dans laquelle la composition contient de plus un ion métallique choisi parmi le cuivre, le magnésium, l'étain, le zinc, le strontium, le calcium et leurs mélanges, dans laquelle le rapport molaire de l'ion métallique au composé ayant des liaisons C-O-P est compris entre de 4:1 et 1:4.

10. Composition orale comprenant;
(a) de 0,001 à 10 pour-cent en poids d'un ou plusieurs composés ayant des liaisons C-O-P choisis parmi le hexakis(dihydrogénophosphate) de myo-inositol, le tétrakis(dihydrogénophosphate) de myo-inositol et leurs sels acceptables sur le plan pharmaceutique.
(b) de 0,001 à 10 pour-cent en poids de chlorure de N-tétradécyl-4-éthylpyridinium;
(c) de 0,05 à 20 pour-cent en poids d'un tensioactif, dans lequel le tensioactif est un ester sorbitan et de poly(oxyde d'éthylène); et
(d) le pourcentage en poids restant est un véhicule oralement acceptable.

11. Procédé pour préparer une composition orale telle que définie dans la revendication 1 comprenant les étapes consistant
(a) à dissoudre le composé ayant des liaisons C-O-P et l'agent assurant la compatibilité dans de l'eau; et
(b) à dissoudre le composé cationique antimicrobien ou une solution de composé cationique antimicrobien dans la solution obtenue dans l'étape (a).
dans lequel on ajuste le pH de la solution à une valeur comprise entre 6 et 8 après l'étape (a) ou l'étape (b).

12. Trousse pour inhiber la formation de tartre dentaire ou de plaque dentaire dans une cavité buccale d'un mammifère comprenant un ou plusieurs composés ayant des liaisons C-O-P dans un véhicule oralement acceptable, dans laquelle le composé ayant des liaisons C-O-P est choisi parmi le hexakis(dihydrogéno phosphate) de myo-inositol, le pentakis(dihydrogéno phosphate) de myo-inositol, le tétrakis(dihydrogéno phosphate) de myo-inositol et leurs sels acceptables sur le plan physiologique; un agent assurant la compatibilité dans un véhicule oralement acceptable, et un ou plusieurs composés cationiques antimicrobiens dans un véhicule oralement acceptable; et un moyen pour maintenir le composé ayant des liaisons C-O-P séparément du composé cationique antimicrobien.

13. Trousse selon la revendication 12, dans laquelle la concentration du composé contenant les liaisons C-O-P est comprise entre 0,001 et 10 pour-cent en poids du véhicule oralement acceptable, la concentration de l'agent assurant la compatibilité est comprise entre 0,001 et 20 pour-cent en poids du véhicule oralement acceptable et la concentration du composé cationique antimicrobien est comprise entre 0,01 et 10 pour-cent en poids du véhicule oralement acceptable.

14. Composition selon l'une des revendications 1 à 10 pour une utilisation dans le traitement du tartre dentaire ou de la plaque dentaire.

15. Utilisation d'une composition selon l'une des revendications 1 à 10 pour la fabrication d'une composition orale pour le traitement du tartre dentaire ou de la plaque dentaire.

16. Utilisation d'un tampon anionique pour stabiliser un mélange d'un composé ayant des liaisons C-O-P tel que défini dans la revendication 1 et un ou plusieurs composés cationiques antimicrobiens pour empêcher une précipitation à partir de solution aqueuse.

17. Utilisation d'un tensioactif pour stabiliser un mélange d'un compasé ayant des liaisons C-O-P tel que défini dans la revendication 1 et un ou plusieurs composés cationiques antimicrobiens pour empêcher une précipitation à partir de solution aqueuse.
